# EUROPEAN PATENT APPLICATION

(11) **EP 1 886 685 A1**
(43) Date of publication of application: **13.02.2008**
(21) Application number: 06291299.3
(22) Date of filing: 11.08.2006
(51) Int. Cl.: A61K 31/57, A61P 31/14, C12N 15/11

(54) **Methods, uses and compositions for modulating replication of hcv through the farnesoid x receptor (fxr) activation or inhibition**

(71) Applicant: INSERM (Institut National de la Santé et de la Recherche Médicale), 75654 Paris Cedex 13 (FR)
(72) Inventor: Andre, Patrice, 69003 Lyon (FR); Lotteau, Vincent, 69390 Vourles (FR); Scholtes, Caroline, 69110 Sainte Foy Lès Lyon (FR)
(74) Representative: Touati, Catherine

(57) **Abstract**

The present invention relates to uses, methods and compositions for modulating replication of viruses of the *Flaviviridae* family, such as hepatitis C virus, through the farnesoid X receptor (FXR) activation or inhibition. More specifically, the invention relates to the use of an antagonist of FXR or an inhibitor of expression thereof for the manufacture of a medicament intended for treating a *Flaviviridae* virus infection in a subject in need thereof. The invention encompasses the use of antagonists of FXR, such as guggulsterone, or use of inhibitors of FXR expression. The invention relates also to a cell culture system allowing the replication of HCV and to methods for diagnosing HCV infection, screening of anti-viral compounds and vaccine or viral protein production.

## Description

The present invention relates to uses, methods and compositions for modulating replication of viruses belonging to the *Flaviviridae* family, such as hepatitis C virus (HCV), through the farnesoid X receptor (FXR) activation or inhibition. More specifically, the invention relates to the use of an antagonist of FXR or an inhibitor of expression thereof for the manufacture of a medicament intended for treating an infection by a member of the *Flaviviridae* family, such as HCV, in a subject in need thereof. The invention relates also to a cell culture system allowing the replication of HCV and its use for diagnosing HCV infections, screening of anti-viral compounds or neutralizing antibodies and for vaccine or viral proteins production.

HCV is a single-stranded positive RNA virus, which belongs to the family of *Flaviviridæ,* genus *Hepacivirus.*

The genome of HCV comprises a single positive-stranded RNA that encodes a polyprotein of about 3010 amino acid residues, flanked at either end by noncoding regions (NCRs). The 5'-NCR and the first part of the region encoding the polyprotein fold into a complex structure of hairpin loops and unpaired regions that can act as an internal ribosome entry site. This means that translation of the virus genome is cap-independent with the initiation of translation directed to the AUG codon at the beginning of the polyprotein rather than the most 5'-terminal AUG. RNA secondary structures have also been described for the 3'-untranslated region, and it is thought that these might play a role in the replication of the virus genome, although there is at present no direct evidence for this. The N-terminus of the polyprotein is comprised of three structural proteins (core, E1 and E2) and release of these proteins from the polyprotein is dependent upon the signal peptidase associated with the cellular endoplasmic reticulum. Release of the six nonstructural proteins (NS2, NS3, NS4A, NS4B, NS5A and NS5B) from the remainder of the polyprotein is mediated by the virus NS2-NS3 and NS3-NS4A proteases.

It is estimated that 170 million patients worldwide and about 1 % of the population in developed countries are chronically infected with hepatitis C virus (HCV) (Global surveillance and control of hepatitis, 1999). The majority (70 to 80%) of acute HCV infections become chronic, some of which progress toward liver cirrhosis or hepatocellular carcinoma.

About 95% of individuals infected with HCV do not develop jaundice during the acute phase of infection, although symptoms may include nausea, anorexia and/or fatigue. Virus RNA can first be detected from 1 to 2 months after exposure, and this is usually accompanied or shortly followed by a rise in blood levels of alanine aminotransferase, a marker for liver damage. Seroconversion occurs from about 3 months after exposure, with reactivity first detected to the NS3 and core antigens. Between 20 and 50% of acutely infected individuals are able to clear virus infection, and this outcome has been correlated with the presence of specific antibody responses to the HVR of the E2 protein and to a CD4+ response to part of the NS3 protein.

Chronic hepatitis C can be clinically silent for many years, although there will often be intermittent elevations in serum alanine aminotransferase levels. Histological examination of liver biopsies from chronically infected individuals usually reveals some signs of liver pathology, although these are often very mild. A typical pathological feature is the presence of lymphoid follicles within the portal tracts, together with periportal inflammation and damage to the bile ducts. In the most extreme cases, the liver becomes cirrhotic with the accumulation of fibrous tissue between hepatocyte nodules eventually leading to liver failure. The extent of liver pathology generally increases with the duration of infection, albeit with a timescale of decades rather than years. Why the rate of progression to disease should vary between individuals is not fully understood. Immunodeficiency can lead to a relatively rapid rate of progression, while there is conflicting evidence for an association between virus genotype and progression, perhaps because of underlying epidemiological associations between virus genotype, the age of acquisition of infection and the duration of infection. Some studies of liver transplant recipients suggest that more severe disease of the transplanted liver occurs in patients infected with HCV of genotype type 1b..

A strong association has been observed between the development of hepatocellular carcinoma and infection with HCV, although this progression is observed in only a small proportion of HCV-infected individuals, and occurs extremely slowly, usually following the development of cirrhosis. The mechanism of oncogenesis in vivo is not known, but the core protein of HCV has limited transforming activity in tissue culture cells and can repress transcription of some cell cycle genes, while the NS5A can act as a transcriptional activator and repress PKR, a cellular protein kinase that is induced by interferon (Gale et al., 1997).

A variety of nonhepatic disorders have been associated with HCV infection, including autoimmune and lymphoproliferative disorders, chronic fatigue, essential type II mixed cryoglobulinaemia, membranoproliferative glomerulonephritis and purpura cutanea tardia. Although these manifestations have been interpreted by some as evidence for extrahepatic replication of HCV, several of these conditions could also be produced indirectly as consequences of chronic liver disease or because of the accumulation of HCV in immune complexes.

Current therapy consists in the association of pegylated interferon (IFN) alpha and ribavirin (1-β-D-ribofuranosyl-1,2,4-triazole-3-carboxamide).

However, the outcome of hepatitis C virus (HCV) infection varies among individuals and the likelihood of sustained response to antiviral treatment depends on viral and host characteristics. Naturally occurring variants of HCV are classified into 6 major genotypes. Viral genotype is one of the main factors associated to therapy response. Indeed, sustained virological response (SVR) is achieved in only 45% of the genotype 1 infected patients, whereas up to 80 % of the genotypes 2 or 3 infected patients reach a SVR (Feld JJ. et al. 2005). On the other side host factors associated to HCV outcome include factors such as age, race, body mass index.

The major site of replication of HCV is thought to be the liver, as high levels of virus RNA are present in liver biopsy material, and both antisense virus genomes and virus nonstructural proteins can be detected in hepatocytes. However, there is controversy about the extent to which virus replicates elsewhere in the body. At first the liver is always reinfected after transplantation. Secondly HCV exists as quasispecies (which are variants found in the same individuals, due to the high mutational rate of the HCV and it seems that the variants originate from different cell types. And finally low-density HCV particules have been described recently as lipo-viro-particles (LVPs).

LVPs are rich in triglycerides, and contain the apolipoproteins B (apoB) and E (apoE) thus resembling very low-density lipoprotein VLDLs. These VLDL-like particles contain HCV RNA, core proteins (Andre P. et al. 2002) and the enveloppe proteins E1 and E2. The nature and the biochemical composition of LVPs suggest that their synthesis could occur in organs specialized in production of apoB-containing lipoproteins. Because HCV replicates in the liver and because VLDLs are synthesized by the liver, this organ is the obvious candidate for LVPs production. However, comparison of RNA quasispecies provides evidence that liver and LVP populations are not identical. In addition to the liver, the intestine is the only other source of apoB lipoproteins and may significantly contribute to the production of these particles. In agreement with the hypothesis that HCV replication takes place in the intestine, HCV proteins have been found in epithelial cells of the small intestine of chronically infected patients (Deforges S. et al. 2004).

Besides this association with lipoproteins as circulating form of the virus, various studies have shown an interaction of HCV with lipid metabolism. Hepatitis C is frequently related with the accumulation of triglycerides promoting liver steatosis (Ramalho F. 2003). Patients with chronic HCV infection show a decrease in total cholesterol load in blood. This observation is not seen in other viral hepatitis (Fischer S. et al. 1996). HCV core and NS5A proteins have been described in cell culture models and in transgenic mice to alter lipid metabolism (Shi ST. et al. 2002). Su et al. (Su Al. et al. 2002) have shown that host genes involved in lipid metabolism are differentially regulated in the early stages of infection in chimpanzees. They also showed that drugs affecting lipid biosynthetic pathways could regulate HCV replication in HCV replicon system. This is in accordance with a recent proteomic study demonstrating that proteins involved in lipid biosynthesis were up regulated in full-length replicon transfected Huh-7 cells whereas proteins involved in fatty acid oxidation were down-regulated (Kapadia SB. et al. 2005). Finally HCV association with lipid rafts has been demonstrated to be critical for efficient replication of this virus (Aizali H. et al. 2004)

Besides the production of lipoproteins, the other pathway controlling lipid homeostasis common to the liver and the intestine is the enterohepatic cycle of bile acids (BAs).

Serum BAs have been recently described as prognostic markers that predict failure to reach SVR (Jorquera F. et al. 2005). High seric concentrations of BAs in chronic hepatitis C are associated with pruritus and advanced pathology. These pruritic patients usually fail to respond to therapy (Lebovics E. et al. 1997). Accordingly, concentrations of serum BAs over 15µM and ferritin higher than 300µg/ml are predictive of non response to therapy (Jorquera F; et al. 2005).

A recent study (Chang KO. et al. 2004) reported that BAs are essential factors for the growth of the porcine enteric calicivirus (PEC), a virus sharing various similarities with HCV (both are viruses with a single-strand plus-sense RNA genome, that can be found in enterocytes).

Now, the present invention provides new methods for up or down regulating the replication of HCV including a new method for the treatment of the HCV infections and a new method for in vitro replication of HCV. The inventors have indeed demonstrated that BAs are involved in HCV RNA replication. Using Huh-7 cell lines transfected transiently with a subgenomic replicon, the inventors have found that treatment of cells with physiological and pathological concentrations of BAs enhanced greatly HCV replication. Furthermore, by using FXR-selective agonists and FXR-specific siRNA the inventors have clearly demonstrated that free BAs were enhancing HCV RNA replication via a FXR-dependent mechanism in Huh-7 cells. Finally, the inventors have shown that the enhancement HCV RNA replication by BAs could be inhibited by the FXR-antagonist guggulsterone. They have shown that this antagonist had beneficial effects to the IFN-treatment.

Therefore a first object of the invention thus relates to the use of an antagonist of farnesoid X receptor (FXR) for the manufacture of a medicament intended for the treatment of an infection by members of the *Flaviviridae* family in a subject in need thereof.

A second object of the invention relates to the use of an antagonist of FXR for the manufacture of a medicament intended for the treatment of an HCV infection or for the treatment of a disease associated with an HCV infection in a subject in need thereof, such as acute or chronic hepatitis C or for the prevention of liver diseases, such as liver fibrosis, liver cirrhosis or hepatocellular carcinoma.

A third object of the invention relates to the use of an inhibitor of FXR expression for the manufacture of a medicament intended for the treatment of an infection by members of the *Flaviviridae* family in a subject in need thereof.

A fourth object of the invention relates to the use of an inhibitor of FXR expression for the manufacture of a medicament intended for the treatment of an HCV infection or for the treatment of a disease associated with an HCV infection in a subject in need thereof, such as acute or chronic hepatitis C or for the prevention of liver diseases, such as liver fibrosis, liver cirrhosis or hepatocellular carcinoma.

A fifth object of the invention relates to a kit for the treatment of an infection by members of the *Flaviviridae* family or for the treatment of an HCV infection or for the treatment of a disease associated with an HCV infection, comprising a medicament comprising an antagonist of FXR or an inhibitor of FXR expression and at least a medicament selected from the group consisting of a medicament comprising interferon-alpha, a medicament comprising a nucleoside analog and a medicament comprising an inhibitor of HCV proteases and/or polymerases.

A sixth object of the invention relates to a cell culture system allowing the replication of HCV, comprising a culture medium for FXR expressing cells, FXR expressing cells and at least one agonist of FXR.

A seventh object of the invention relates to the use of the cell culture system as above described for diagnosing HCV infections, or screening of anti-viral compounds, or producing HCV viral particles or HCV viral proteins or producing of anti-HCV vaccines

An eighth object of the invention relates to an in vitro method for diagnosing an HCV infection in a subject wherein said method comprises the steps consisting of :
a) providing a culture of FXR expressing cells
b) incubating said culture of FXR expressing cells with a biological sample obtained from the subject,
c) incubating said culture of FXR expressing cells with at least one agonist of FXR prior to, after or simultaneously with step (b).
d) culturing said cells for a time sufficient for permitting HCV replication
e) detecting the level of HCV replication
wherein the detection of an HCV replication is indicative that said subject is infected with HCV.

### Definitions:

The term "hepatitis C virus" or "HCV" is used herein to define a viral species of which pathogenic strains cause hepatitis C, also known as non-A, non-B hepatitis. Therefore the term "HCVs" denotes hepatitis C viruses.

The term "HCV particle" denotes the virus particles that contain HCV structural proteins and are observable under electron microscopy. HCV particles vary in size, between 30 to 60 nm in diameter. In addition, HCV particles display significant heterogeneity in buoyant density on sucrose density-gradient centrifugation, ranging from low (<1.07 g/ml) to high (1.25 g/ml) density. The heterogeneity of particle density has been attributed to the variability in size, non-enveloped nucleocapsid particles, and association with antibodies or B-lipoproteins.

As used herein, the term "HCV viral RNA", which includes HCV RNA, refers to RNA from the HCV genome, fragments thereof, transcripts thereof, and mutant sequences derived therefrom.

The expression "replication of the HCV" designates the molecular process or processes leading to the synthesis of HCV RNA and/or viral particles.

A "coding sequence" or a sequence "encoding" an expression product, such as an RNA, polypeptide, protein, or enzyme, is a nucleotide sequence that, when expressed, results in the production of that RNA, polypeptide, protein, or enzyme, i.e., the nucleotide sequence encodes an amino acid sequence for that polypeptide, protein or enzyme. A coding sequence for a protein may include a start codon (usually ATG) and a stop codon.

The term "gene" means a DNA sequence that codes for or corresponds to a particular sequence of amino acids which comprise all or part of one or more proteins or enzymes, and may or may not include regulatory DNA sequences, such as promoter sequences, which determine for example the conditions under which the gene is expressed. Some genes, which are not structural genes, may be transcribed from DNA to RNA, but are not translated into an amino acid sequence. Other genes may function as regulators of structural genes or as regulators of DNA transcription. In particular, the term gene may be intended for the genomic sequence encoding a protein, i.e. a sequence comprising regulator, promoter, intron and exon sequences.

As used herein, the term "oligonucleotide" refers to a nucleic acid, generally of at least 10, preferably at least 12, more preferably at least 15, and still preferably at least 20 nucleotides, preferably no more than 100 nucleotides, still preferably no more than 70 nucleotides, and which is hybridizable to a genomic DNA, cDNA, or mRNA.

As used herein, references to specific proteins (e.g., FXR) can include a polypeptide having a native amino acid sequence, as well as variants and modified forms regardless of their origin or mode of preparation. A protein that has a native amino acid sequence is a protein having the same amino acid sequence as obtained from nature (e.g., a naturally occurring FXR). Such native sequence proteins can be isolated from nature or can be prepared using standard recombinant and/or synthetic methods. Native sequence proteins specifically encompass naturally occurring truncated or soluble forms, naturally occurring variant forms (e.g., alternatively spliced forms), naturally occurring allelic variants and forms including postranslational modifications. A native sequence protein includes proteins following post-translational modifications such as glycosylation, or phosphorylation, or other modifications of some amino acid residues.

Variants refer to proteins that are functional equivalents to a native sequence protein that have similar amino acid sequences and retain, to some extent, one or more activities of the native protein. Variants also include fragments that retain activity. Variants also include proteins that are substantially identical (e.g., that have 80, 85, 90, 95, 97, 98, 99%, sequence identity) to a native sequence. Such variants include proteins having amino acid alterations such as deletions, insertions and/or substitutions. A "deletion" refers to the absence of one or more amino acid residues in the related protein. The term "insertion" refers to the addition of one or more amino acids in the related protein. A "substitution" refers to the replacement of one or more amino acid residues by another amino acid residue in the polypeptide. Typically, such alterations are conservative in nature such that the activity of the variant protein is substantially similar to a native sequence protein (see, e.g., Creighton (1984) Proteins, W.H. Freeman and Company). In the case of substitutions, the amino acid replacing another amino acid usually has similar structural and/or chemical properties. Insertions and deletions are typically in the range of 1 to 5 amino acids, although depending upon the location of the insertion, more amino acids can be inserted or removed. The variations can be made using methods known in the art such as site-directed mutagenesis (Carter, et al. (1986) Nucl. Acids Res. 13:4331; Zoller et al. (1987) Nucl. Acids Res. 10:6487), cassette mutagenesis (Wells et al. (1985) Gene 34:315), restriction selection mutagenesis (Wells, et al. (1986) Philos. Trans. R. Soc. London SerA 317:415), and PCR mutagenesis (Sambrook et al., Molecular Cloning: A Laboratory Manual, 3rd edition, Cold Spring Harbor Press, N.Y., (2001)).

Two amino acid sequences are "substantially homologous" or "substantially similar" when greater than 80 %, preferably greater than 85 %, preferably greater than 90 % of the amino acids are identical, or greater than about 90 %, preferably grater than 95 %, are similar (functionally identical). Preferably, the similar or homologous sequences are identified by alignment using, for example, the GCG (Genetics Computer Group, Program Manual for the GCG Package, Version 7, Madison, Wisconsin) pileup program, or any of sequence comparison algorithms such as BLAST, FASTA, etc.

The term "expression" when used in the context of expression of a gene or nucleic acid refers to the conversion of the information, contained in a gene, into a gene product. A gene product can be the direct transcriptional product of a gene (e.g., mRNA, tRNA, rRNA, antisense RNA, ribozyme, structural RNA or any other type of RNA) or a protein produced by translation of a mRNA. Gene products also include messenger RNAs which are modified, by processes such as capping, polyadenylation, methylation, and editing, and proteins (e.g., FXR) modified by, for example, methylation, acetylation, phosphorylation, ubiquitination, SUMOylation, ADP-ribosylation, myristilation, and glycosylation.

An "inhibitor of expression" refers to a natural or synthetic compound that has a biological effect to inhibit the expression of a gene. Therefore, an "inhibitor of FXR expression" denotes a natural or synthetic compound that has a biological effect to inhibit the expression of FXR gene.

A "receptor" or "receptor molecule" is a soluble or membrane bound/associated protein or glycoprotein comprising one or more domains to which a ligand binds to form a receptor-ligand complex. By binding the ligand, which may be an agonist or an antagonist the receptor is activated or inactivated and may initiate or block pathway signalling.

The term "FXR" refers to the farnesoid X receptor, which is a nuclear receptor that is activated by supraphysiological levels of farnesol (Forman et al., Cell, 1995,81,687-693). FXR, is also known as NR1H4, retinoid X receptor-interacting protein 14 (RIP14) and bile acid receptor (BAR). FXR is a member of the nuclear receptor superfamily of ligand-activated transcription factors and forms, with retinoid X receptor (RXR), a heterodimer receptor crucial for bile acid homeostasis (Forman et al. 1995). FXR is expressed in various tissues including the liver, kidney, intestine, colon, ovary, and adrenal gland (Forman et al. 1995). Containing a conserved DNA-binding domain (DBD) and a C-terminal ligand-binding domain (LBD), FXR binds to and becomes activated by a variety of naturally occurring bile acids (BAs), including the primary bile acid chenodeoxycholic acid (CDCA) and its taurine and glycine conjugates (Makishima et al., 1999; Parks et al., 1999; Wang et al., 1999). Upon activation, the FXR-RXR heterodimer binds the promoter region of target genes and regulates the expression of several genes involved in bile acid homeostasis. Hepatic FXR target genes fall into two main groups (Edwards PA. et al. 2002, Kapadia SB. Et al. 2005). The first group functions to decrease hepatic bile acids concentrations by increasing export and decreasing their synthesis. The second group of FXR target genes such as the phospholipid transport protein PLTP and apolipoproteins modulates lipoprotein levels in the serum and decreases plasma triglyceride concentration. FXR plays also an important role in controlling liver growth and regeneration by delivering homeotrophic signals in response to variations of BA concentrations (Huang W. et al. 2006) For a more detailed list of FXR-regulated genes, see, e.g., WO 03/016288, pages 22-23. US patent 6,005, 086 discloses the nucleic acid sequence coding for a mammalian FXR protein. The human polypeptide sequences for FXR are deposited in nucleotide and protein databases under accession numbers NM_005123, Q96R11, NP_005114 AAM53551, AAM53550, AAK60271.

By "ligand" or "receptor ligand" is meant a natural or synthetic compound which binds a receptor molecule to form a receptor-ligand complex. The term ligand includes agonists, antagonists, and compounds with partial agonist/antagonist action.

An "agonist" or "receptor agonist" is a natural or synthetic compound which binds the receptor to form a receptor-agonist complex by activating said receptor and receptor-agonist complex, respectively, initiating a pathway signalling and further biological processes.

By "antagonist" or "receptor antagonist" is meant a natural or synthetic compound that has a biological effect opposite to that of an agonist. An antagonist binds the receptor and blocks the action of a receptor agonist. An antagonist is defined by its ability to block the actions of an agonist.

Therefore, the term "agonist of FXR" denotes a substance that binds FXR and activates its functions such as induction of transcription of genes under the dependence of FXR response elements in their promoters.

Therefore, the term "antagonist of FXR" denotes a substance that inhibits the activity of FXR such as the induction of transcription caused by FXR. More specifically, it is a substance that inhibits the binding of FXR and a coactivator of the receptor.

The term "small organic molecule" refers to a molecule of a size comparable to those organic molecules generally used in pharmaceuticals. The term excludes biological macromolecules (e. g. , proteins, nucleic acids, etc.). Preferred small organic molecules range in size up to about 5000 Da, more preferably up to 2000 Da, and most preferably up to about 1000 Da.

By "purified" or "isolated" is meant, when referring to a polypeptide (i.e. interferon) or a nucleotide sequence, that the indicated molecule is present in the substantial absence of other biological macromolecules of the same type. The term "purified" as used herein preferably means at least 75% by weight, more preferably at least 85% by weight, still preferably at least 95% by weight, and most preferably at least 98% by weight, of biological macromolecules of the same type are present. An "isolated" nucleic acid molecule which encodes a particular polypeptide refers to a nucleic acid molecule which is substantially free of other nucleic acid molecules that do not encode the subject polypeptide; however, the molecule may include some additional bases or moieties which do not deleteriously affect the basic characteristics of the composition.

As used herein, the term "subject" denotes a mammal, such as a rodent, a feline, a canine, and a primate. Preferably a subject according to the invention is a human.

The term "cell culture system" refers to a system that allow the in vitro culture of cells.

### Therapeutic methods and uses

The present invention provides for uses, methods and compositions (such as pharmaceutical compositions) for treating an infection by a virus belonging to the *Flaviviridae* family in a subject in need thereof.

In one embodiment the member of the *Flaviviridae* family may be a member of the *Flavivirus* genus such as the Japanese encephalitis virus group, including Japanese encephalitis virus and West Nile Virus. Alternatively it may be a member of the Yellow fever virus group. Alternatively it may be a member of the Pestivirus genus, such as Bovine viral diarrhea virus (BVDV-1 and/or BVDV-2), Classical swine fever virus, Border disease virus. Alternatively, the *Flaviviridae* members may belong to the Hepacivirus genus such as the hepatitis G virus (HGV).

In a particular embodiment, the invention provides uses, methods and compositions for treating a hepatitis C virus (HCV) infection. More specifically the present invention provides for uses, methods and compositions for treating acute or chronic C hepatitis, or diseases associated with a Hepatitis C virus infection such as autoimmune and lymphoproliferative disorders, chronic fatigue, essential type II mixed cryoglobulinaemia, membranoproliferative glomerulonephritis and purpura cutanea tardia. The present invention also encompasses uses, methods and composition for the prevention and treatment of liver diseases, such as liver fibrosis, liver cirrhosis or hepatocellular carcinoma.

The inventors have indeed demonstrated that BAs are involved in HCV RNA replication via a FXR dependent signaling pathway. Therefore, the inhibition of FXR signaling or expression represents a promising tool for the treatment of Hepatitis C infections. Moreover, the invention provides for new uses, methods and compositions that are suitable for achieving a sustained viral response or for increasing the rate of sustained viral response after current therapies.

The term "sustained viral response" (SVR; also referred to as a "sustained response" or a "durable response"), as used herein, refers to the response of an individual to a treatment regimen for HCV infection, in terms of serum HCV titer. Generally, a "sustained viral response" refers to no detectable HCV RNA (e. g. , less than about 500, less than about 200, or less than about 50 genome copies per milliliter serum) found in the patient's serum for a period of at least about one month, at least about two months, at least about three months, at least about four months, at least about five months, or at least about six months following cessation of treatment.

Thus an object of the invention is the use of an antagonist of farnesoid X receptor (FXR) for the manufacture of a medicament intended for the treatment of an infection by members of the *Flaviviridae* family in a subject in need thereof.

Another object of the invention is the use of an antagonist of FXR for the manufacture of a medicament intended for the treatment of an HCV infection or for the treatment of a disease associated with an HCV infection in a subject in need thereof, such as acute or chronic hepatitis C or for the prevention of liver diseases, such as liver fibrosis, liver cirrhosis or hepatocellular carcinoma.

In one embodiment, an antagonist of FXR may be a small organic molecule. International Patent Applications WO02064125, WO0220463, WO03015771, WO03016288, WO2004046068, US patent application US2004176426 and US patent US6906057 disclose small organic molecules that may be used as antagonists of FXR according to the invention (each of these documents are incorporated in their entirety by reference).

A specific example of small organic molecule that can be used according to the present invention may be the guggulsterone. Extracts of the resin of the guggul tree (*Commiphora mukul*) were indeed shown as lowering LDL (low-density lipoprotein) cholesterol levels in humans. The plant sterol guggulsterone [4,17(20)-pregnadiene-3,16-dione] is the active agent in this extract, and was shown as a highly efficacious antagonist of FXR (Urizar NL et al. 1996; Wu J. et al. 2002). Guggulsterone exists in two isomer forms : Z- and E-guggulsterone. International Patent Application WO2004094450 and US patent application US2005085452 describe processes for preparation of both isomers. The Z isomer form (4,17(20)-trans-pregnadiene-3,16-dione) has the structure of formula :

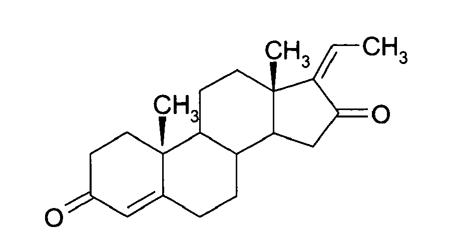

The E isomer form (4,17(20)-cis-pregnadiene-3,16-dione) has the structure of formula :

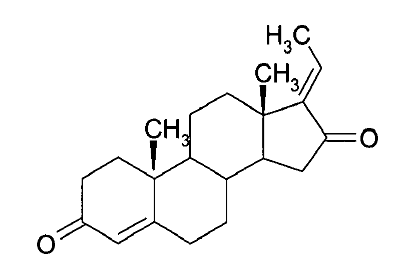

Another specific example of a small organic molecule that can be used according to the invention may be the 3-β-hydroxy-5,16-pregnadien-20-one (also known as 80-574, Wu J. et al. 2002), which is an analog of guggulsterone. Said compound has the structure of formula :

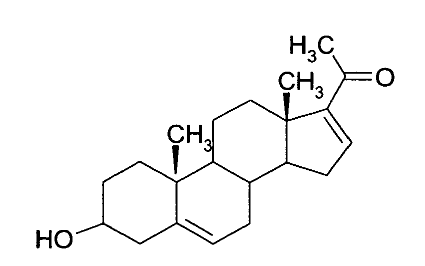

In a preferred embodiment of the invention, said antagonist of FXR is 4,17(20)-trans-pregnadiene-3,16-dione or 4,17(20)-cis-pregnadiene-3,16-dione.

Another object of the invention is the use of an inhibitor of FXR expression for the manufacture of a medicament intended for the treatment of an infection by members of the *Flaviviridae* family in a subject in need thereof.

Another object of the invention is the use of an inhibitor of FXR expression for the manufacture of a medicament intended for the treatment of an HCV infection or for the treatment of a disease associated with an HCV infection in a subject in need thereof, such as acute or chronic hepatitis C or for the prevention of liver diseases, such as liver fibrosis, liver cirrhosis or hepatocellular carcinoma..

In a preferred embodiment of the invention, said inhibitor of FXR expression is a siRNA, an antisense oligonucleotide or a ribozyme.

Inhibitors of FXR expression for use in the present invention may be based on antisense oligonucleotide constructs. Anti-sense oligonucleotides, including anti-sense RNA molecules and anti-sense DNA molecules, would act to directly block the translation of FXR mRNA by binding thereto and thus preventing protein translation or increasing mRNA degradation, thus decreasing the level of FXR proteins, and thus activity, in a cell. For example, antisense oligonucleotides of at least about 15 bases and complementary to unique regions of the mRNA transcript sequence encoding FXR can be synthesized, e.g., by conventional phosphodiester techniques and administered by e.g., intravenous injection or infusion. Methods for using antisense techniques for specifically inhibiting gene expression of genes whose sequence is known are well known in the art (e.g. see U.S. Pat. Nos. 6,566,135; 6,566,131; 6,365,354; 6,410,323; 6,107,091; 6,046,321; and 5,981,732).

Small inhibitory RNAs (siRNAs) can also function as inhibitors of FXR expression for use in the present invention. FXR gene expression can be reduced by contacting the tumor, subject or cell with a small double stranded RNA (dsRNA), or a vector or construct causing the production of a small double stranded RNA, such that FXR expression is specifically inhibited (i.e. RNA interference or RNAi). Methods for selecting an appropriate dsRNA or dsRNA-encoding vector are well known in the art for genes whose sequence is known (e.g. see Tuschi, T. et al. (1999); Elbashir, S. M. et al. (2001); Hannon, GJ. (2002); McManus, MT. et al. (2002); Brummelkamp, TR. et al. (2002); U.S. Pat. Nos. 6,573,099 and 6,506,559; and International Patent Publication Nos. WO 01/36646, WO 99/32619, and WO 01/68836).

Ribozymes can also function as inhibitors of FXR expression for use in the present invention. Ribozymes are enzymatic RNA molecules capable of catalyzing the specific cleavage of RNA. The mechanism of ribozyme action involves sequence specific hybridization of the ribozyme molecule to complementary target RNA, followed by endonucleolytic cleavage. Engineered hairpin or hammerhead motif ribozyme molecules that specifically and efficiently catalyze endonucleolytic cleavage of FXR mRNA sequences are thereby useful within the scope of the present invention. Specific ribozyme cleavage sites within any potential RNA target are initially identified by scanning the target molecule for ribozyme cleavage sites, which typically include the following sequences, GUA, GUU, and GUC. Once identified, short RNA sequences of between about 15 and 20 ribonucleotides corresponding to the region of the target gene containing the cleavage site can be evaluated for predicted structural features, such as secondary structure, that can render the oligonucleotide sequence unsuitable. The suitability of candidate targets can also be evaluated by testing their accessibility to hybridization with complementary oligonucleotides, using, e.g., ribonuclease protection assays.

Both antisense oligonucleotides and ribozymes useful as inhibitors of FXR expression can be prepared by known methods. These include techniques for chemical synthesis such as, e.g., by solid phase phosphoramadite chemical synthesis. Alternatively, anti-sense RNA molecules can be generated by in vitro or in vivo transcription of DNA sequences encoding the RNA molecule. Such DNA sequences can be incorporated into a wide variety of vectors that incorporate suitable RNA polymerase promoters such as the T7 or SP6 polymerase promoters. Various modifications to the oligonucleotides of the invention can be introduced as a means of increasing intracellular stability and half-life. Possible modifications include but are not limited to the addition of flanking sequences of ribonucleotides or deoxyribonucleotides to the 5' and/or 3' ends of the molecule, or the use of phosphorothioate or 2'-O-methyl rather than phosphodiesterase linkages within the oligonucleotide backbone.

International patent applications WO03044167 and WO2004030750 provide for antisense oligonucleotides and methods using thereof for modulating the FXR expression.

Antisense oligonucleotides siRNAs and ribozymes of the invention may be delivered in vivo alone or in association with a vector. In its broadest sense, a "vector" is any vehicle capable of facilitating the transfer of the antisense oligonucleotide siRNA or ribozyme nucleic acid to the cells and preferably cells expressing FXR. Preferably, the vector transports the nucleic acid to cells with reduced degradation relative to the extent of degradation that would result in the absence of the vector. In general, the vectors useful in the invention include, but are not limited to, plasmids, phagemids, viruses, other vehicles derived from viral or bacterial sources that have been manipulated by the insertion or incorporation of the the antisense oligonucleotide siRNA or ribozyme nucleic acid sequences. Viral vectors are a preferred type of vector and include, but are not limited to nucleic acid sequences from the following viruses: retrovirus, such as moloney murine leukemia virus, harvey murine sarcoma virus, murine mammary tumor virus, and rouse sarcoma virus; adenovirus, adeno-associated virus; SV40-type viruses; polyoma viruses; Epstein-Barr viruses; papilloma viruses; herpes virus; vaccinia virus; polio virus; and RNA virus such as a retrovirus. One can readily employ other vectors not named but known to the art.

Preferred viral vectors are based on non-cytopathic eukaryotic viruses in which non-essential genes have been replaced with the gene of interest. Non-cytopathic viruses include retroviruses (e.g., lentivirus), the life cycle of which involves reverse transcription of genomic viral RNA into DNA with subsequent proviral integration into host cellular DNA. Retroviruses have been approved for human gene therapy trials. Most useful are those retroviruses that are replication-deficient (i.e., capable of directing synthesis of the desired proteins, but incapable of manufacturing an infectious particle). Such genetically altered retroviral expression vectors have general utility for the high-efficiency transduction of genes in vivo. Standard protocols for producing replication-deficient retroviruses (including the steps of incorporation of exogenous genetic material into a plasmid, transfection of a packaging cell lined with plasmid, production of recombinant retroviruses by the packaging cell line, collection of viral particles from tissue culture media, and infection of the target cells with viral particles) are provided in KRIEGLER (A Laboratory Manual," W.H. Freeman C.O., New York, 1990) and in MURRY ("Methods in Molecular Biology," vol.7, Humana Press, Inc., Cliffton, N.J., 1991).

Preferred viruses for certain applications are the adeno-viruses and adeno-associated viruses, which are double-stranded DNA viruses that have already been approved for human use in gene therapy. The adeno-associated virus can be engineered to be replication deficient and is capable of infecting a wide range of cell types and species. It further has advantages such as, heat and lipid solvent stability; high transduction frequencies in cells of diverse lineages, including hemopoietic cells; and lack of superinfection inhibition thus allowing multiple series of transductions. Reportedly, the adeno-associated virus can integrate into human cellular DNA in a site-specific manner, thereby minimizing the possibility of insertional mutagenesis and variability of inserted gene expression characteristic of retroviral infection. In addition, wild-type adeno-associated virus infections have been followed in tissue culture for greater than 100 passages in the absence of selective pressure, implying that the adeno-associated virus genomic integration is a relatively stable event. The adeno-associated virus can also function in an extrachromosomal fashion.

Other vectors include plasmid vectors. Plasmid vectors have been extensively described in the art and are well known to those of skill in the art. See e.g., SANBROOK et al., "Molecular Cloning: A Laboratory Manual," Second Edition, Cold Spring Harbor Laboratory Press, 1989. In the last few years, plasmid vectors have been used as DNA vaccines for delivering antigen-encoding genes to cells in vivo. They are particularly advantageous for this because they do not have the same safety concerns as with many of the viral vectors. These plasmids, however, having a promoter compatible with the host cell, can express a peptide from a gene operatively encoded within the plasmid. Some commonly used plasmids include pBR322, pUC18, pUCI9, pRC/CMV, SV40, and pBlueScript. Other plasmids are well known to those of ordinary skill in the art. Additionally, plasmids may be custom designed using restriction enzymes and ligation reactions to remove and add specific fragments of DNA. Plasmids may be delivered by a variety of parenteral, mucosal and topical routes. For example, the DNA plasmid can be injected by intramuscular, intradermal, subcutaneous, or other routes. It may also be administered by intranasal sprays or drops, rectal suppository and orally. It may also be administered into the epidermis or a mucosal surface using a gene-gun. The plasmids may be given in an aqueous solution, dried onto gold particles or in association with another DNA delivery system including but not limited to liposomes, dendrimers, cochleate and microencapsulation.

Another object of the invention relates to a method for the treatment of an HCV infection or a disease associated with an HCV infection, such as acute or chronic hepatitis C or for the prevention of liver diseases, such as liver fibrosis, liver cirrhosis or hepatocellular carcinoma comprising the administration of a therapeutically effective amount of at least one antagonist of FXR or inhibitor of FXR expression to a subject in need thereof.

In the context of the invention, the term "treating" or "treatment", as used herein, means reversing, alleviating, inhibiting the progress of, or preventing the disorder or condition to which such term applies, or one or more symptoms of such disorder or condition.

According to the invention, the term "subject" or "patient" and "subject in need thereof" or "patient in need thereof", is intended for a human or non-human mammal infected or likely to be infected with a hepatitis C virus.

By a "therapeutically effective amount" of the antagonist or inhibitor of expression as above described is meant a sufficient amount of the antagonist or inhibitor of expression to treat a hepatitis C virus infection at a reasonable benefit/risk ratio applicable to any medical treatment. It will be understood, however, that the total daily usage of the compounds and compositions of the present invention will be decided by the attending physician within the scope of sound medical judgment. The specific therapeutically effective dose level for any particular patient will depend upon a variety of factors including the disorder being treated and the severity of the disorder; activity of the specific compound employed; the specific composition employed, the age, body weight, general health, sex and diet of the patient; the time of administration, route of administration, and rate of excretion of the specific compound employed; the duration of the treatment; drugs used in combination or coincidential with the specific polypeptide employed; and like factors well known in the medical arts. For example, it is well within the skill of the art to start doses of the compound at levels lower than those required to achieve the desired therapeutic effect and to gradually increase the dosage until the desired effect is achieved. However, the daily dosage of the products may be varied over a wide range from 0.01 to 1,000 mg per adult per day. Preferably, the compositions contain 0.01, 0.05, 0.1, 0.5, 1.0, 2.5, 5.0, 10.0, 15.0, 25.0, 50.0, 100, 250 and 500 mg of the active ingredient for the symptomatic adjustment of the dosage to the patient to be treated. A medicament typically contains from about 0.01 mg to about 500 mg of the active ingredient, preferably from 1 mg to about 100 mg of the active ingredient. An effective amount of the drug is ordinarily supplied at a dosage level from 0.0002 mg/kg to about 20 mg/kg of body weight per day, especially from about 0.001 mg/kg to 7 mg/kg of body weight per day.

Any of the above treatment regimens can be administered to individuals who have been diagnosed with an HCV infection. Any of the above treatment regimens can be administered to individuals who have failed previous treatment for HCV infection (treatment failure patients).

"Treatment failure patients" as used herein generally refers to HCV-infected patients who failed to respond to previous therapy for HCV (referred to as "non-responders") or who initially responded to previous therapy, but in whom the therapeutic response was not maintained (referred to as "relapsers"). The previous therapy generally can include treatment with IFN-alpha monotherapy or IFN-alpha combination therapy, where the combination therapy may include administration of IFN-alpha and an antiviral agent such as ribavirin.

Individuals who have been clinically diagnosed as infected with HCV are of particular interest in many embodiments. Individuals who are infected with HCV are identified as having HCV RNA in their blood, and/or having anti-HCV antibody in their serum. Such individuals include anti-HCV ELISA-positive individuals. Such individuals may also, but need not, have elevated serum alanine aminotransferase (ALT) levels. Individuals who are clinically diagnosed as infected with HCV include naive individuals (e. g. , individuals not previously treated for HCV, particularly those who have not previously received IFN-alpha-based and/or ribavirin-based therapy) and individuals who have failed prior treatment for HCV ("treatment failure" patients).

Treatment failure patients include non-responders, namely individuals in whom the HCV titer was not significantly or sufficiently reduced by a previous treatment for HCV, e. g. , a previous IFN-alpha monotherapy, a previous IFN-alpha and ribavirin combination therapy, or a previous pegylated IFN-alpha and ribavirin combination therapy). Treatment failure patients include relapsers, namely individuals who were previously treated for HCV, such as individuals, who received a previous IFN-alpha monotherapy, a previous IFN-alpha and ribavirin combination therapy, or a previous pegylated IFN-alpha and ribavirin combination therapy, whose HCV titer decreased, and subsequently increased.

In other embodiments, the invention thus provides for a method for treating an HCV infection comprising administering a subject in need thereof with a therapeutically effective amount of an antagonist of FXR or inhibitor of FXR expression as above described, wherein said subject is a non responder or relapser patient.

In one embodiment, the invention relates to the use of an antagonist of FXR or an inhibitor of FXR as described above for the treatment of a subject undergoing a treatment with interferon-alpha.

In other embodiment, methods of the invention further comprises administering to the subject an effective amount of interferon alpha (IFN-alpha).

Any known IFN-alpha can be used in the instant invention. The term "interferon-alpha" or "IFN-alpha" as used herein refers to a family of related polypeptides that inhibit viral replication and cellular proliferation and modulate immune response. The term "IFN-alpha" includes naturally occurring IFN-alpha; synthetic IFN-alpha ; derivatized IFN-alpha (e. g. , PEGylated IFN-alpha, glycosylated IFN-alpha, and the like); and analogs of naturally occurring or synthetic IFN-alpha ; essentially any IFN-alpha that has antiviral properties, as described for naturally occurring IFN-alpha. Suitable interferons alpha include, but are not limited to, naturally-occurring IFN-alpha (including, but not limited to, naturally occurring IFN-alpha2a, IFN-alpha2b) ; recombinant interferon alpha-2b such as Intron-A interferon available from Schering Corporation, Kenilworth, N. J. ; recombinant interferon alpha-2a such as Roferon interferon available from Hoffmann-La Roche, Nutley, N. J. ; recombinant interferon alpha-2C such as Berofor alpha 2 interferon available from Boehringer Ingelheim Pharmaceutical, Inc. , Ridgefield, Conn.; interferon alpha- n1, a purified blend of natural interferon alphas such as Sumiferon available from Sumitomo, Japan or as Wellferon interferon alpha-n1 (INS) available from the Glaxo-Wellcome Ltd., London, Great Britain; and interferon alpha-n3 a mixture of natural interferon alphas made by Interferon Sciences and available from the Purdue Frederick Co. , Norwalk, Conn. , under the Alferon Tradename.

The term "IFN-alpha" also encompasses derivatives of IFN-alpha that are derivatized (e. g. , are chemically modified) to alter certain properties such as serum half-life. As such, the term "IFN-alpha" includes glycosylated IFN-alpha ; IFN-alpha derivatized with polyethylene glycol ("PEGylated IFN-alpha"); and the like. PEGylated IFN-alpha, and methods for making same, is discussed in, e. g., U. S. Patent Nos. 5,382, 657; 5,981, 709; and 5,951, 974. PEGylated IFN-alpha encompasses conjugates of PEG and any of the above-described IFN-alpha molecules, including, but not limited to, PEG conjugated to interferon alpha-2a (Roferon,Hoffman La-Roche, Nutley, N. J.), interferon alpha 2b (Intron, Schering-Plough, Madison, N. J. ), interferon alpha-2c (Berofor Alpha, Boehringer Ingelheim, Ingelheim, Germany); and consensus interferon as defined by determination of a consensus sequence of naturally occurring interferons alpha (Infergen (InterMune, Inc. , Brisbane, Calif.).

In another embodiment, the invention relates to the use of an antagonist of FXR or an inhibitor of FXR as described above for the treatment of a subject undergoing a treatment with a nucleoside analog.

In other embodiments, methods of the invention further comprises administering to the subject an effective amount of a nucleoside analog for achieving a sustained viral response. Said nucleoside analog may be ribavirin or derivatives thereof.

Ribavirin (1-β-D-ribofuranosyl-1,2,4-triazole-3-carboxamide), is a nucleoside analog available from ICN Pharmaceuticals, Inc. , Costa Mesa,Calif., and is described in the Merck Index, compound No. 8199, Eleventh Edition. Its manufacture and formulation is described in U. S. Pat. No. 4,211, 771.

The invention also contemplates use of derivatives of ribavirin as those described in US patent 6,277, 830, or International Patent Application WO2006067606. Other derivatives include Levovirin which is the L-enantiomer of ribavirin, or Viramidine which is a 3-carboxamidine derivative of ribavirin.

In another embodiment, the invention relates to the use of an antagonist of FXR or an inhibitor of FXR as described above for the treatment of a subject undergoing a treatment with interferon-alpha and a nucleoside analog.

In other embodiments, methods of the invention further comprises administering an effective amount of interferon alpha (IFN-alpha) and a nucleoside analog as those above described.

In another embodiment, the invention relates to the use of an antagonist of FXR or an inhibitor of FXR as described above for the treatment of a subject undergoing a treatment with an inhibitor of HCV proteases and/or polymerases.

In other embodiments, methods of the invention further comprises administering to the subject an effective amount of an inhibitor of HCV polymerases. Such inhibitors include, but are not limited to a compound as disclosed in U. S. Patent No. 6,479, 508; a compound as disclosed in any of International Patent Application WO03010140; WO03007945, WO03010141, WO0147883, a dinucleotide analog as disclosed in Zhong et al. (2003); a benzothiadiazine compound as disclosed in Dhanak et al.(2002); an NS5B inhibitor as disclosed in W002100846 or WO 0200851, W00185172, WO 02098424, WO 0006529, WO 0206246, WO 03000254, or EP 1 256,628 A2.

In other embodiments, methods of the invention further comprises administering to the subject an effective amount of an inhibitor of HCV proteases. Inhibitors of NS3 protease include, but are not limited to, a compound as disclosed in Internationa Patent Applications WO03066103 WO2004103996 or WO2004093915. HCV NS3 protease inhibitor peptide analogs include any compound disclosed in Patent Applications GB 2,337,262; JP10298151; JP 11126861; JP 11292840; JP 2001-103993; U.S. Pat. No. 6,159,938; U.S. Pat. No. 6,187,905; WO 97/43310; WO 98/17679; WO 98/22496; WO 98/46597; WO 98/46630; WO 99/38888; WO 99/50230; WO 99/64442; WO 99/07733; WO 99/07734; WO 00/09543; WO 00/09558; WO 00/20400; WO 00/59929; WO 00/31129; WO 01/02424; WO 01/07407; WO 01/16357; WO 01/32691; WO 01/40262; WO 01/58929; WO 01/64678; WO 01/74768; WO 01/77113; WO 01/81325; WO 02/08187; WO 02/08198; WO 02/08244; WO 02/08251; WO 02/08256; WO 02/18369; WO 02/60926 and WO 02/79234. Inhibitors of HCV NS3 protease have been also described in WO 03/064456, WO 03/064416 , WO 02/060926, WO 03/053349, WO 03/099316, WO 03/099274, WO 2004/032827, and. WO 2004/043339. Acyl sulfamide inhibitors of the HCV NS3 protease have also been described in WO 2005/046712 or WO2006000085. Inhibitors of NS3-NS4A protease are described in Patent Applications WO2005007681 WO2005035525 or WO2005028502.

In other embodiments, methods of the invention further comprises administering to the subject an effective amount of a statin compound. Statins, which are 3-hydroxy-3-methylglutaryl coenzyme A (HMG-CoA) reductase inhibitors may be potentially useful as anti-HCV reagents (Ikeda M. et al. 2006, Kapadia SB. et al. 2005, Ye J. et al. 2003).

In another embodiment, the invention relates to the use of an antagonist of FXR or an inhibitor of FXR as described above for the treatment of a subject undergoing a treatment with interferon-alpha, a nucleoside analog and an inhibitor of HCV proteases and/or polymerases.

Another object of the present invention is a kit for the treatment of an infection by members of the *Flaviviridae* family or for the treatment of an HCV infection or for the treatment of a disease associated with an HCV infection, comprising a medicament comprising an antagonist of FXR or an inhibitor of FXR expression and at least a medicament selected from the group consisting of a medicament comprising interferon-alpha, a medicament comprising a nucleoside analog and a medicament comprising an inhibitor of HCV proteases and/or polymerases.

### Screening methods :

In further embodiment the antagonists of FXR according to the invention may be obtained by any screening method well known in the art.

For example, a method for the in vitro screening of antagonists of FXR may comprise the steps consisting in (a) contacting cells transfected with, and expressing, DNA encoding for FXR with a ligand known to bind specifically to FXR (e.g. a natural ligand such as a BA); (b) contacting the cells of step (a) with a candidate compound; (c) comparing the binding of the ligand known to bind to FXR in the presence of said candidate compound, to the binding of ligand known to bind to FXR in the absence of said candidate compound, and (d) selecting positively the candidate compound that reduces the binding of the ligand known to bind to FXR.

Other suitable in vitro screening method according to the invention can be carried out using labeled candidate compounds which are then incubated with a polypeptide that has a FXR ligand binding domain (e.g., a full-length FXR polypeptide). Labels include radioisotopes, immunochemicals, fluorophores, and the like. Those of skill in the art will recognize a variety of ways of separating the bound labeled candidate therapeutic agent from the free labeled candidate therapeutic agent. The affinity of the labeled candidate therapeutic agent for a FXR polypeptide can be calculated using standard ligand binding methods.

Another type of a screening method according to the invention may consist in testing the ability of a test compound to modulate binding of FXR to a ligand for FXR. These can be conducted, for example, as a direct binding assay with a labeled FXR ligand in the presence of a candidate therapeutic agent. The assays involve placing the test compound into an assay mixture that includes at least a ligand binding domain of a FXR polypeptide and a ligand for FXR. The effect on binding of the FXR ligand to FXR is determined. A test compound that decreases the amount of labeled FXR ligand that is bound to a FXR polypeptide or a polypeptide that has a FXR ligand binding domain, is of interest for future screening for its ability to inhibit replication of HCV. A suitable technique that can be used in said screening methods may be the Homogeneous Time Resolved Fluorescence (HTRF) assay, such as described in document WO 00/01663 or US6,740,756.

Ligands that are suitable for use in the in vitro screening methods of the invention include, but are not limited to, bile acids and related compounds such as CDCA (chenodeoxycholic acid), GCDCA (glycochenodeoxycholic acid), TCDCA (taurochenodeoxycholic acid), GCA (glycocholic acid), TCA (taurocholic acid), DCA (deoxycholic acid), LCA (lithocholic acid), DHCA (dehydrocholic acid), UDCA (ursodeoxycholic acid) and CA (cholic acid). Additional bile acids and other ligands are described in, for example, Makishima et al. (1999) Science 284:1362-1365. The assays can also employ coactivators and corepressors with which FXR interacts.

International patent applications WO0040965, WO02064125 WO03030612 describe in vitro screening methods that may be suitable for identifying antagonists of FXR according to the invention.

Furthermore International patent application WO2004046323 provides compositions comprising the ligand binding domain (LBD) of a farnesoid X receptor (FXR) in crystalline form. Said document further provides in silico methods of using this structural information to screen antagonists of FXR.

The candidate compounds that have been positively selected at the end of any one of the embodiments of the in vitro or in silico screening which has been described previously in the present specification may be subjected to further selection steps in view of further assaying its anti-HCV biological properties. For this purpose, the candidate compounds that have been positively selected with the general in vitro screening methods as above described may be further selected for their ability to inhibit the replication of HCV. In a particular embodiment, the ability to inhibit the replication of HCV is assayed by using the replicon system as described in example. Briefly, replicons are biscistronic RNAs which contains the firely luciferase (*Photinus pyralis*) reporter downstream of the HCV IRES and the sequence encoding for the polyprotein NS3 to NS5B downstream of the EMCV IRES. Said replicons are then transfected into cells expressing FXR such as Huh7 cells. The up-regulation of HCV RNA replication thus results in the increase of the luciferase activity in cells transfected with replicon systems.

Therefore, the invention provides for a method for the in cellulo screening of compounds that inhibit the replication of HCV, wherein said methods comprises:
a) providing a FXR antagonist that have been identified according to the in vitro screening methods as above described
b) providing a cell transfected with a replicon system as above described
c) bringing into contact the FXR antagonist of step a) with cells of step b)
d) detecting the luciferase activity of said replicon system
e) comparing said luciferase activity with luciferase activity obtained in the absence of said FXR antagonist
wherein a decrease in the luciferase activity obtained in the presence of said FXR antagonist comparing to the luciferase activity obtained in the absence of said FXR antagonist is indicative that said FXR antagonist can inhibit the replication of HCV.

### Pharmaceutical compositions:

The antagonist or inhibitor of expression of the invention may be combined with pharmaceutically acceptable excipients, and optionally sustained-release matrices, such as biodegradable polymers, to form therapeutic compositions.

"Pharmaceutically" or "pharmaceutically acceptable" refers to molecular entities and compositions that do not produce an adverse, allergic or other untoward reaction when administered to a mammal, especially a human, as appropriate. A pharmaceutically acceptable carrier or excipient refers to a nontoxic solid, semi-solid or liquid filler, diluent, encapsulating material or formulation auxiliary of any type.

In the pharmaceutical compositions of the present invention for oral, sublingual, subcutaneous, intramuscular, intravenous, transdermal, local or rectal administration, the active principle, alone or in combination with another active principle, can be administered in a unit administration form, as a mixture with conventional pharmaceutical supports, to animals and human beings. Suitable unit administration forms comprise oral-route forms such as tablets, gel capsules, powders, granules and oral suspensions or solutions, sublingual and buccal administration forms, aerosols, implants, subcutaneous, transdermal, topical, intraperitoneal, intramuscular, intravenous, subdermal, transdermal, intrathecal and intranasal administration forms and rectal administration forms.

Preferably, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The pharmaceutical forms suitable for injectable use include sterile aqueous solutions or dispersions; formulations including sesame oil, peanut oil or aqueous propylene glycol ; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi.

Solutions comprising compounds of the invention as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

The antagonist or inhibitor of expression of the invention can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

Sterile injectable solutions are prepared by incorporating the active polypeptides in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution and either added to 1000 ml of hypodermoclysis fluid or injected at the proposed site of infusion. Some variation in dosage will necessarily occur depending on the condition of the subject being treated. The person responsible for administration will, in any event, determine the appropriate dose for the individual subject.

The antagonist or inhibitor of expression of the invention may be formulated within a therapeutic mixture to comprise about 0.0001 to 1.0 milligrams, or about 0.001 to 0.1 milligrams, or about 0.1 to 1.0 or even about 10 milligrams per dose or so. Multiple doses can also be administered.

In addition to the compounds of the invention formulated for parenteral administration, such as intravenous or intramuscular injection, other pharmaceutically acceptable forms include, e.g. tablets or other solids for oral administration ; liposomal formulations ; time release capsules ; and any other form currently used.

### Systems for the in vitro replication of HCV :

The inventors have indeed demonstrated that BAs are involved in HCV RNA replication via a FXR dependent signaling pathway. Therefore, without whishing to be bound by any particular theory, the inventors believe that this observation explains why natural hepatocytes or hepatoma cell lines cannot be infected in vitro by the HCV virus. Therefore, the invention provides for methods that are suitable for achieving the in vitro replication of HCV in large amounts.

Therefore, an object of the invention relates to a cell culture system allowing the replication of HCV, comprising a culture medium for FXR expressing cells, FXR expressing cells and at least one agonist of FXR.

The term "FXR expressing cell" denotes a cell that expresses FXR naturally or not.

In a general manner, FXR is expressed in various tissues including the liver, kidney, intestine, colon, ovary, and adrenal gland (Forman et al. 1995). Therefore FRX expressing cell include any cell that has been isolated from the above mentioned tissues. In a particular embodiment, FXR expressing cells include isolated hepatocytes and intestinal cells. In other particular embodiment FXR expressing cells consist in primary tissue cultures of liver or intestine. FXR expressing cells may be also isolated from cell line derived form the above described tissue. In a preferred embodiment of the invention, said expressing FXR cells are chosen among cell monolayers of the human hepatoma cell line Huh7 (Nakabayashi H. et al. 1982) or Huh7-Lunet (Quinkert D. et al. 2005) or HepG2.

In other embodiment, FXR expressing cells may be cells that have been transfected with the gene encoding for FXR.

In other embodiment, FXR expressing cells may be cells that have been transfected in a way such that it overexpress FXR, which means that the level of FXR expressed by said cells is at least 1.2, preferably at least 1.5 and more preferably at least 2 times higher the the level of FXR expressed in a cell naturally expressing FXR.

The term "transfection" means the introduction of a foreign nucleic acid into a cell, thus the term "transfection" includes in the present invention the terms "transduction" and "infection". The introduced gene or sequence may also be called a "cloned" or "foreign" gene or sequence, may include regulatory or control sequences, such as start, stop, promoter, signal, secretion, or other sequences used by a cellular molecular machinery. A host cell that receives and expresses introduced DNA or RNA has been "transfected" and is a "transfectant" or a "recombinant cell".

Recombinant DNA techniques are well-known in the art. For example, the gene encoding for FXR or fragment thereof can be incorporated into expression vectors. Then such vectors are introduced into suitable eukaryotic host cells that will express the desired polypeptide.

A wide variety of host/expression vector combinations can be employed in expressing the nucleic acids encoding for FXR. Useful expression vectors that can be used include, for example, segments of chromosomal, non-chromosomal and synthetic DNA sequences. Suitable vectors include, but are not limited to, derivatives of SV40 and pcDNA and known bacterial plasmids such as col El, pCR1, pBR322, pMal-C2, pET, pGEX, pMB9 and derivatives thereof, plasmids such as RP4, phage DNAs such as the numerous derivatives of phage I such as NM989, as well as other phage DNA such as M13 and filamentous single stranded phage DNA; yeast plasmids such as the 2 microns plasmid or derivatives of the 2 microns plasmid, as well as centomeric and integrative yeast shuttle vectors; vectors useful in eukaryotic cells such as vectors useful in insect or mammalian cells; vectors derived from combinations of plasmids and phage DNAs, such as plasmids that have been modified to employ phage DNA or the expression control sequences and the like.

Consequently, mammalian and typically human cells may be transfected by the nucleic acid or recombinant vector as defined herein. Examples of suitable cells include, but are not limited to, VERO cells, HELA cells such as ATCC No. CCL2, CHO cell lines such as ATCC No. CCL61, COS cells such as COS-7 cells and ATCC No. CRL 1650 cells, W138, BHK, 3T3 such as ATCC No. CRL6361, A549, PC12, K562 cells, 293T cells, Sf9 cells such as ATCC No. CRL1711 and Cv1 cells such as ATCC No. CCL70.

FXR agonists may be chosen among natural agonists of FXR such as bile acids and related compounds that include CDCA (chenodeoxycholic acid), GCDCA (glycochenodeoxycholic acid), TCDCA (taurochenodeoxycholic acid), GCA (glycocholic acid), TCA (taurocholic acid), DCA (deoxycholic acid), LCA (lithocholic acid), DHCA (dehydrocholic acid), UDCA (ursodeoxycholic acid) and CA (cholic acid). Additional bile acids and other agonists are described in, for example, Makishima et al. (1999). FXR agonists also include but are not limited to those described in Patent Applications, WO03090745, WO03080803, WO03016288 , WO2004046162, WO2004048349, WO2005082925, WO2005097097, WO2005092328, US2006128764, US2005080064 and JP2005281155. FXR agonists also include but are not limited to cholesterol (Cho), 22-hydroxycholesterol (22-OH-cho), 25-hydroxycholesterol (25-OH-cho), pregnenolone, progesterone, dexamethasone (DXM), (E)-[(tetrahydrotetramethylnaphthalenyl)propenyl]benzoic acid (TTNPB), farnesol, and retinoic acid (RA).

In a preferred embodiment of the invention, the agonist of FXR is chenodeoxycholic acid, deoxycholic acid, lithocholic acid, dehydrocholic acid, ursodeoxycholic acid, cholic acid, farnesol or (E)-[(tetrahydrotetramethylnaphthalenyl)propenyl]benzoic acid.

In an embodiment of the invention, the cell culture system as described above further comprises HCV viruses. HCV viruses may be isolated from various origins and any genotypes. HCV viruses can be isolated from a subject infected with HCV. In another embodiment of the invention, the cell culture system comprises FXR expressing cells that are transfected with replicative HCV viral materials, such as HCV replicons. Replicons may be constructed without limitation from any HCV genotypes or biological samples. Said replicons may be those described in the example as hereinafter described.

One object of the invention is the use of the cell culture system as described above, for diagnosing HCV infections, or screening of anti-viral compounds, or producing HCV viral particles or HCV viral proteins or producing anti-HCV vaccines.

The invention also provides an in vitro method for diagnosing an HCV infection in a subject wherein said method comprises the steps consisting of:
a) providing a culture of FXR expressing cells
b) incubating said culture of FXR expressing cells with a biological sample obtained from the subject,
c) incubating said culture of FXR expressing cells with at least one agonist of FXR prior to, after or simultaneously with step (b).
d) culturing said cells for a time sufficient for permitting HCV replication
e) detecting the level of HCV replication
wherein the detection of an HCV replication is indicative that said subject is infected with HCV.

The biological sample can be derived without limitation from blood, serum, plasma or from a sample isolated during a biopsy.

Detection of the HCV replication can be performed by any known technique in the art. Such techniques may include anti-HCV ELISA assay (Enzyme Linked ImmunoSorbent Assay), which tests for HCV proteins. Testing for HCV replication by amplification tests RNA (e.g. polymerase chain reaction or PCR, branched DNA assay) may be used. The synthesis of the RNAs of the HCV may be indeed analysed by RT-PCR in a single step using a device designed for real time PCR or by hybridization of the RNAs on filters using HCV-specific radioactive probes. For instance, the isolated RNA may be subjected to coupled reverse transcription and amplification, such as reverse transcription and amplification by polymerase chain reaction (RT-PCR), using specific oligonucleotide primers that enable amplification of HCV genome. Then an direct sequencing may be performed to determine the genotype of HCV that has infected said subject

A positive result indicates the presence of infectious viruses in the samples and therefore an active infection and a potential for spread of the infection and or/the development of liver disease such as liver fibrosis, cirrhosis and hepatocellular carcinoma.

The invention also provides another in vitro method for diagnosing an HCV infection in a subject. This alternative method allows the detection of antibodies against HCV in a biological sample from a subject, said method comprising the steps of
a) providing a culture of FXR expressing cells,
b) incubating said culture of FXR expressing cells with HCV viruses or transfecting said cells with replicative HCV viral materials,
c) incubating said culture of FXR expressing cells with at least one agonist of FXR prior to, after or simultaneously with step (b),
d) culturing said cells to produce HCV viral proteins,
e) incubating said culture of FXR expressing cells with a biological sample obtained from the subject, under conditions that permit interaction of HCV-specific antibodies in the sample with the HCV protein(s),
f) detecting binding of the antibodies in the sample to the HCV-derived protein(s),
wherein said binding is indicative of the presence of HCV infection in the subject from which the sample was derived.

In the foregoing method, the biological sample can be derived without limitation from blood, serum, plasma, blood cells, lymphocytes, or liver tissue biopsy. Techniques for isolating proteins and cellular fractions useful in the foregoing diagnostic methods are also well known in the art.

The cell culture system as above described may also be useful for producing high amounts or viral particles and/or HCV derived proteins. HCV particles may be indeed isolated from cell cultures obtained according to the method hereafter described (or from their culture medium) under conditions that permit virus particle formation.

The invention also relate to a method for producing HCV viral particles or HCV viral proteins, comprising the steps consisting of:
a) providing a culture of FXR expressing cells,
b) incubating said culture of FXR expressing cells with HCV viruses or transfecting said cells with replicative HCV viral materials,
c) incubating said culture of FXR expressing cells with at least one agonist of FXR prior to, after or simultaneously with step (b),
d) culturing said cells.

In a specific embodiment the viral particles produced with the cell culture system as above described may be useful for producing an attenuated recombinant vaccine that can be administered to an individual to produce an anti-viral immune response.

Alternatively, isolated HCV-derived proteins expressed by the cell culture system as above described may represent starting materials for producing an HCV vaccine.

Still alternatively, the isolated HCV-derived proteins and/or viral particles expressed by the cell culture system may be useful for producing antibodies directed against said HCV-derived proteins and/or viral particles in particular antibodies with neutralizing properties. Amount of HCV-derived proteins isolated from the cell cultures may be administered to an animal, for producing anti-HCV antibodies. A further method for producing antibodies to HCV comprises screening a human antibody library for reactivity with HCV-derived proteins and selecting a clone from the library that expresses a reactive antibody. Alternatively, monoclonal anti-HCV antibodies can be produced in hybridoma cell lines using techniques well known in the art.

Antibodies produced by the above mentioned method may be useful in therapeutic aim for treating HCV infection. Said antibodies may be useful for diagnosing HCV infection in a subject. Therefore said antibodies may be used for producing kits for diagnosing an HCV infection in a subject. HCV-specific antibodies prepared according to the invention can be used to detect HCV presence and/or propagation in various biological samples.

Since the cell culture system of the invention is not limited by the use of a particular genotype, said system may be therefore useful for screening and/or manufacture of new vaccines and/or antiviral molecules, in particular for the screening of molecules active vis-a-vis one of the viral cycle stages. Said culture system may be useful for screening in vitro for agents capable of modulating HCV infection and/or replication and/or virion assembly.

The invention relates to a method for screening for antiviral molecules, comprising the steps consisting of:
a) providing a culture of FXR expressing cells,
b) incubating said culture of FXR expressing cells with HCV viruses or transfecting said cells with replicative HCV viral materials,
c) incubating said culture of FXR expressing cells with at least one agonist of FXR prior to, after or simultaneously with step (b).
d) incubating said culture of FXR expressing cells with a candidate compound prior to, after or simultaneously with steps (b) or (c),
e) measuring the level of HCV replication and/or HCV-associated protein expression and/or HCV viral particles.

A decrease in the level of HCV replication and/or HCV-associated protein expression and/or HCV viral particles observed in the presence of the candidate compound, relative to the level HCV replication and/or HCV-associated protein expression and/or HCV viral particles observed in its absence, is indicative of the inhibitory activity of the candidate compound.

For example inhibition of viral particles formation can be detected microscopically (performed directly or after immunostaining); and changes in infectivity of generated HCV virus particles can be assayed by isolating them from the cell culture medium and applying to naive cells or a susceptible animal model.

In a specific embodiment, the cell culture system of the present invention provides a convenient system for high-throughput initial screening of potential anti-HCV therapeutics. Such high-throughput screening system involves applying test compounds to the cell culture system (growing, e.g., in 96- or 324-well microtiter plates) followed by measuring changes (e.g., using multi-plate readers or scanners) in HCV replication and/or HCV-associated protein expression and/or HCV particles production.

According to invention, candidate therapeutic compounds include without limitation small molecules, inhibitory peptides, inhibitory (e.g., transdominant-negative) proteins, antibodies and in particular neutralizing antibodies, ribozymes, and antisense nucleic acids.

As disclosed herein, the anti-HCV therapeutic compounds identified using the in vitro screening methods as above described can be further characterized for their ability to affect HCV propagation using secondary screens in susceptible animal models. Based on the tropism of the HCV, a preferred small animal model of the present invention is a tree shrew Tupaia belangeri chinensis. A preferred large animal model is a chimpanzee. Test animals will be treated with the candidate compounds that produced the strongest inhibitory effects (control animals would not be treated, and, if available, a positive control could also be employed). A compound that protects animals from infection by virus and/or inhibits viral propagation leading to pathogenicity, would be an attractive candidate for development of an agent for treatment/prevention of HCV infection. In addition, the animal models provide a platform for pharmacokinetic and toxicology studies.

The invention will further be illustrated in view of the following figures and example.

### FIGURES :

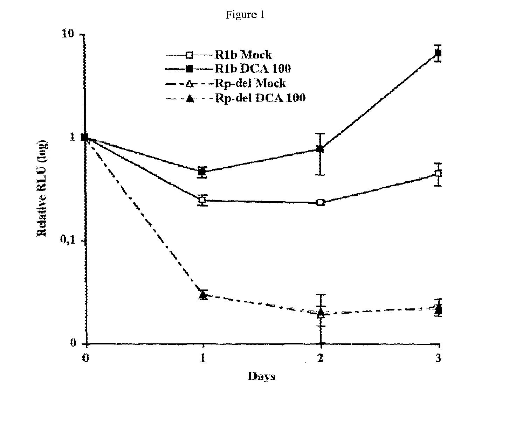

**Figure 1: Enhancement of HCV replicon 1b replication by DCA : Short-term effect of DCA :** Huh7 cells were transfected by electroporation either with the replicative replicon R1 b (square) or with the defective replicon Rp-del (triangle, dashed lines). 4h after electroporation, cells were treated (closed dots) or not (open dots) with DCA (100µM). After indicated days of DCA treatment, cells were lysed and luciferase activity (relative light units RLU) was measured. Results are given as relative ratios of luciferase activity at 4h and are means of triplicate ± S.D.

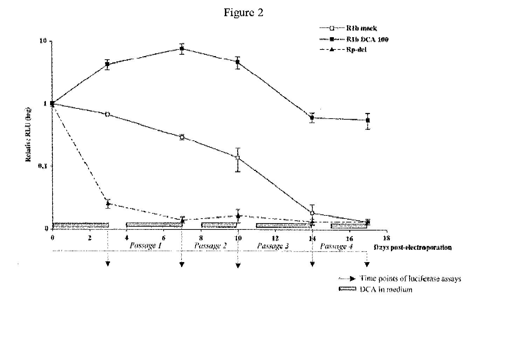

**Figure 2: Enhancement of HCV replicon 1b replication by DCA : Long-term effect of DCA :** Huh7 cells were treated as described in A. DCA was present in culture medium all along the culture, except for 24h after each passage as indicated by the hatched bars on top of the abscissa. Cells were passaged 72h posttransfection, then on days 7, 10 and 14 at a 1:3 dilution as indicated by the dashed lines. Cells were harvested at given time points to determine luciferase activity (arrows). Results are expressed as previously described in figure 1.

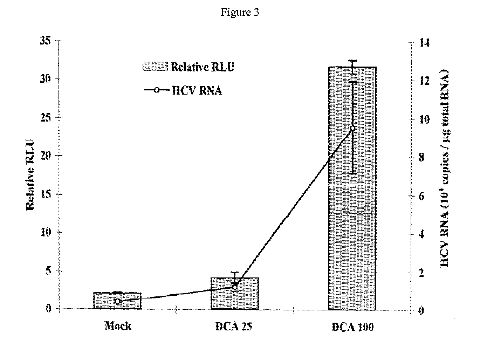

**Figure 3: Correlation between luciferase activity and HCV negative strand RNA copie number :** Huh7-Lunet cells were transfected by electroporation with R1 b. 4h after electroporation, cells were treated or not with DCA (25µM and 100µM). Cells were passaged once at a dilution of 1:3. 6 days after transfection, one part of the cells was lysed for luciferase activity measurement and the other part was used to quantify RNA levels. HCV RNA levels were determined by RQ-PCR and normalised using total RNA quantitation.

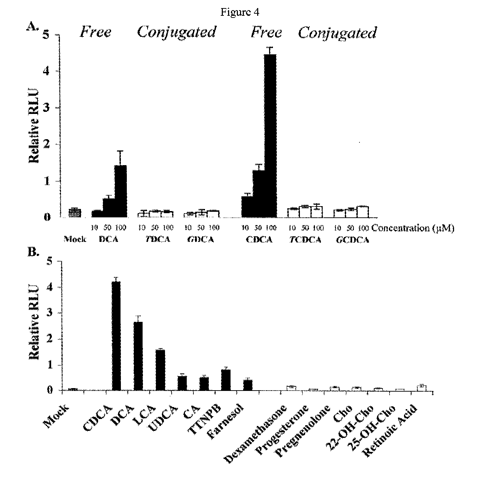

**Figure 4: Ligands of FXR including BA up-regulate HCV replication : A.** Dose-response effect of free and conjugated BAs on subgenomic HCV RNA replication. Huh7 cells were transfected by electroporation with the replicative replicon R1 b. 4h after the electroporation, Huh7 cells were treated for 72h with 10, 50 or 100 µM of the free bile acids DCA (closed bars, left panel) and CDCA (closed bars, right panel) and their glyco- G or tauro- T conjugated derivatives (open bars). Untreated cells (with R1 b alone) are shown as a dashed bar. Results are expressed as previously described in Figure 1. **B.** Only farnesoid X receptor (FXR) agonists upregulate HCV RNA replication. Cells were treated for 72h with 100µM of the indicated bile acid [chenodeoxycholic acid (CDCA), deoxycholic acid (DCA), lithocholic acid (LCA), cholic acid (CA), ursodeoxycholic acid (UDCA)] or with one of the following steroids: cholesterol (Cho), dexamethasone (DXM), pregnenolone, farnesol, TTNPB at 10µM each; 22-hydroxycholesterol (22-OH-Cho), progesterone at 5µM; 25-hydroxycholesterol (25-OH-cho) and 9-cis retinoic acid at 1µM. FXR agonists are shown as closed bars whereas other NR1 agonists are shown as open bars. Results are expressed as previously described in Figure 1.

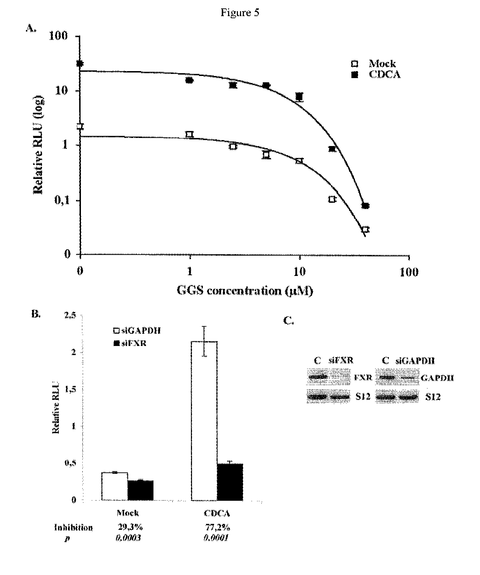

**Figure 5: FXR inhibition decreases HCV RNA replication : A.** Guggulsterone inhibits basal and BAs induced replication of HCV. Huh7-Lunet cells were transfected with R1 b. 4h after electroporation, cells were treated or not with 100µM of CDCA and with different concentrations of guggulsterone (from 0,01µM up to 20µM) for 72h. Results are expressed as previously described in Figure 1. **B.** Silencing of FXR abolishes the upregulation of HCV replication by CDCA in Huh7 cells. Huh7 cells were transfected with 60 nM small interfering RNA (siRNA) duplexes specific for FXR (closed bars) or GAPDH (open bars). After overnight incubation, cells were transfected with R1 b and treated with CDCA (100 µM; right panels) or not (left panel, R1 b alone). Luciferase activity was determined 72h after the electroporation. Results are expressed as previously described in Figure 1. Values under the graph indicate the percentage of inhibition of the replication due to FXR inactivation. The significance of these inhibitions rates are indicated below by the p values of a Student's t-test. **C.** RT-PCR analysis of FXR, GAPDH and RibS12 expression. Total RNA was extracted from Huh7 cells 48h after siRNA transfection RT-PCR for FXR, GAPDH and RibS12 were performed as described in material and methods. RT-PCR for RibS12 was performed as an internal control. RT-PCR products (362 pb for FXR, 199 pb for GAPDH and 338 pb for RibS12) were detected by staining with ethidium bromide after 3% agarose gel electrophoresis.

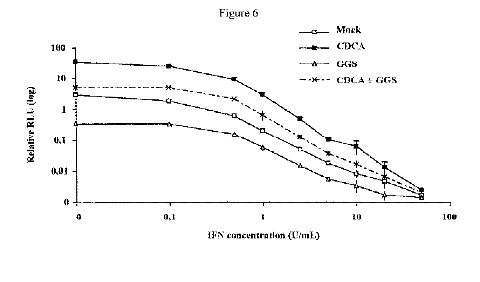

**Figure 6: IFN and FXR modulators act independently on HCV RNA replication :** Transfected Huh7-Lunet cells were treated for 72h with increasing doses of IFN-alpha2b. Conditions used were R1 b alone (open scare), R1 b plus GGS at 10µM (open triangle), R1b plus CDCA at 100µM (closed scare) and R1 b plus CDCA plus GGS at the same concentrations as before (cross dashed line). Results are expressed as previously described in Figure 1.

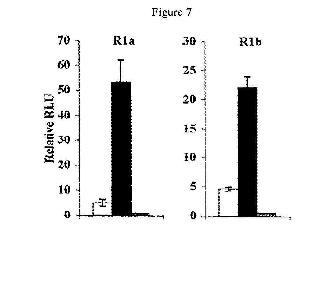

**Figure 7: Differential activity of FXR on different HCV genotypes:** Huh7-Lunet cells were transfected either with genotype 1 a replicon (R1 a, left panel), or with genotype 1 b replicon (R1 b, right panel). 4h after electroporation, cells were treated either with 100µM CDCA (cloded bars) or with 10µM GGS (dashed bars); mock treated cells are shown as open bars. Results are expressed as described in Figure 1.

### EXAMPLE:

### Materials and methods

**Materials :** Apart from the IFNa-2b that was from Schering-Plough, all chemicals used in this study were purchased from Sigma (Saint-Quentin, France).

The BAs tested included cholic acid (CA), chenodeoxycholic acid (CDCA), glycochenodeoxycholic acid (GCDCA), taurochenodeoxycholic acid (TCDCA), deoxycholic acid (DCA), glycodeoxycholic acid (GDCA), taurodeoxycholic acid (TDCA), lithocholic acid (LCA) and ursodeoxycholic acid (UDCA).

The nuclear receptor agonists used were cholesterol (Cho), 22-hydroxycholesterol (22-OH-cho), 25-hydroxycholesterol (25-OH-cho), pregnenolone, progesterone, dexamethasone (DXM), (E)-[(tetrahydrotetramethylnaphthalenyl)propenyl]benzoic acid (TTNPB), farnesol, and retinoic acid (RA).

Guggulsterone (GGS) [trans-4,17(20)-pregnadiene-3,16-dione] was used as FXR antagonist.

All compounds were prepared as 10 mM stock solutions in water, ethanol or dimethylsulfoxyde according to their solubility. These stock solutions were diluted extemporaneously to 10X as working solutions in complete DMEM and added into the cell cultures 4h after electroporation to obtain the final desired concentration.

**Antibodies :** NS4A (2E3C2) (Ferrieu-Weisbuch C. et al. 2006), NS5A (4F3H2) (Deforges, S. et al. 2004.) monoclonal antibodies were kindly provided by Biomerieux . FITC-conjugated F(ab')2 goat anti-mouse IgG (Jackson Immunoresearch) was used as secondary antibody.

**Cell culture :** Cell monolayers of the human hepatoma cell line Huh7 (Nakabayashi H. et al. 1982) or Huh7-Lunet (Quinkert D. et al. 2005) were routinely grown at 37°C in a humidified 5% CO₂ atmosphere in 'complete medium' which refers to Dulbecco's modified minimal essential medium (DMEM) supplemented with 2 mM L-Glutamine, 1 mM HEPES, 1 % non essential amino acids, 50 U of penicilline, 50 µg of streptomycin (all from Life Technologies, Cergy-Pontoise, France) and 10% fetal calf serum (PAN Biotech GmbH, Aidenbach, Germany). Naive cells were passaged twice a week using 0,05% trypsin - 0,02% EDTA (Life Technologies) and seeding at a dilution of 1:3 to 1:5.

**Replicons :** Three subgenomic replicons were used in this study: two replicative forms called R1a and R1 b according to their genotype origin and a derivative of the R1 b replicon with a mutation in the RNA-dependant RNA polymerase (Rp-del) which is thus unable to replicate and was used as a negative control in all experiments. They are bicistronic RNAs which contains the firefly luciferase (Photinus pyralis) reporter gene downstream of the HCV IRES and the sequence coding for the polyprotein NS3 to NS5B downstream of the encephalomyocarditis virus (EMCV) IRES. The plasmid constructs pFK-I-341-PI-luc/NS3-3'/ET (27) (for R1b), pFK-1-341-PI-luc/NS3-3'/GND (for Rp-del) and pFK-I-341-PI-Iuc/NS3-3'/H77/DR (R1a) used to generate the replicons were obtained from Ralf Bartenschlager (University of Heidelberg, Germany) (Kronke J. et al. 2004 ;Lohmann V. et al. 2003)

To generate transcripts of HCV replicons, plasmid DNAs were first restricted by Asel (New Englands Biolabs, Saint Quentin, France) and Scal (Roche Diagnostics, Meylan, France). Then, after extraction with phenol-chloroform and ethanol precipitation, DNA was dissolved in RNase-free water and used for in vitro transcription by the RiboMax large Scale RNA production system T7 (Promega). The reaction mixture contained 80 mM HEPES (pH 7.5), 24 mM MgCl2, 2 mM spermidine, 40mM dithiothreitol (DTT), 3 mM each nucleoside triphosphate, 5 µg restricted plasmid DNA and 10 U T7 RNA polymerase. Transcription was carried out during 4h at 37°C and terminated by 20 min incubation at 37°C with 2U of RNase-free DNase (Promega) per µg of plasmid DNA.

Transcripts were extracted and purified with acidic phenol-chloroform, then precipitated with isopropanol and dissolved in RNase-free water. RNA integrity was checked by denaturing agarose gel electrophoresis and the concentrations were determined by measurement of the optical density at 260 nm.

**RNA transfection :** The replicons were transfected into Huh7 cells by electroporation.

Subconfluent monolayers of Huh7 cells were detached from the culture dish by trypsin treatment and rinsing with complete DMEM. The cells were washed once with phosphate buffer saline (PBS), counted and resuspended at 10⁷ cells per mL in Cytomix buffer (Van den Hoff, MJ. Et al. 1992). 400 µL of the cell suspension was mixed by gentle pipetting with 8 µg RNA (R1 a, R1 b or Rp-del) and transferred into an electroporation cuvette with a gap-width of 0,4 cm (BioRad). The mixture was immediately subjected to one electric pulse at 270 V, 950 µF and maximum resistance using a Gene Pulser System (BioRad).

After electroporation the 400µL of each cell suspension was diluted in 9 mL complete DMEM and pooled together before being seeded onto 24-well plates (1 mL per well).

**Luciferase Assay :** Cells were washed with PBS and scraped off the plate with 80µL of 1X Luciferase Cell Culture Lysis Reagent (Promega). 40pL of the lysate were transferred to a 96-well plate and mixed with 200 µL of Luciferase Assay Reagent (Promega). Luminescence expressed as relative light units (RLU) was measured immediately on a scintillation counter (Top Count NXT, Perkin Elmer) for 10 s.

The data are averages from triplicate cultures. According to Krieger et al., luciferase activity from cells harvested 4h after electroporation was used to determine the transfection efficiency (Krieger N. et al. 2001). The values are expressed as relative ratios of the values obtained 4h after electroporation. Unless otherwise stated, the RLU values were determined 72h after transfection when cells were confluent.

**HCV RNA quantification :** Electroporated cells were treated or not with 25 and 100 µM of DCA for 6 days. Total RNAs were extracted from the cultured cells using the RNeasy Mini kit (Qiagen S.A., Courtaboeuf, France) according to the manufacturer's instructions and quantified by spectrophotometry at 260 nm. Quantification of the negative strand of the Rp was performed by real-time quantitative RT-PCR (RQ-PCR) of the 5' HCV untranslated region as previously described (Komurian-Pradel F. et al. 2004). Briefly, 4µL of total RNA was reverse transcribed using the Thermoscript™ reverse transcriptase kit (Invitrogen) with the primer tag-RC1 (SEQ lD No 1, 5' GGC CGT CAT GGT GGC GAA TAA GTC TAG CCA TGG CGT TAG TA 3') specific for the negative strand of HCV 5'UTR; after a denaturing step of 8 min at 70°C followed by 5 min at 4°C, RNA template was incubated at 60°C for 1 h with 7,5U of Thermoscript™ reverse transcriptase and 20U of RNaseOUT. Transcription was terminated by 5 min incubation at 95°C followed by 5 min at 4°C. 2µL of the synthesized cDNA was subjected to real-time quantitative PCR on a LightCycler apparatus (Roche Diagnostics, Meylan, France) using 5 pmol of the primer pair tagRC1 and RC21 (SEQ ID No 2, 5'CTC CCG GGG CAC TCG CAA GC 3') in a final volume of 20 µL with the LightCycler FastStart DNA Master SYBR Green I kit. Thermocycling conditions were as follow: after an initial denaturation step at 95°C for 120 s, the PCR consisted of 45 cycles of denaturation (95°C for 2 s), annealing (60°C for 5 s) and extension (72°C for 15 s). For each step, the temperature transition rate was 20°C s⁻¹ and the fluorescence monitoring was done after each elongation step. Specificity provided by the selected primers could be confirmed by melting curve analysis of the amplified products. Quantification was carried out using an external standard curve.

**Small interfering RNA experiments :** Huh7 cells were plated in 100 mm petri dishes at 5.10⁶ cells per dish. After overnight adherence, small interfering RNA (siRNA) transfections were performed for 4h in OptiMEM medium (Life Technologies) containing 10µg/mL Lipofectamine 2000™ (Invitrogen) and 100nM siRNA. The medium was then replaced with fresh complete DMEM. After overnight incubation, R1 b was transfected by electroporation following the protocol described earlier. siRNA duplexes specific for FXR (siFXR) were purchased from Dharmakon (Lafayette, CO) as a SMARTpool™ (M-003414-00-0005 human NR1H4). A siRNA specific for glyceraldehyde-3-phosphate dehydrogenase GAPDH, purchased from Ambion (Austin, TX) was used as control to test non-specific effects.

The efficiency of RNA knockdown was checked by RT-PCR using Thermoscript reverse transcriptase kit (Invitrogen) according to the manufacturer's protocol. Total RNA was isolated 4h after electroporation using TRlzol reagent (Invitrogen) PCR primers were 5'GCAGCCTGAAGAGTGGTACTCTC3' (SEQ ID No 3), 5'CATTCAGCCAACATTCCCATCTC3' (SEQ ID No 4) for FXR, 5'GGAAGGTGAAGGTCGGAGTC3' (SEQ lD No 5), 5'CACAAGCTTCCCGTTCTCAG3' (SEQ lD No 6) for GAPDH and 5'GGAGGTGTAATGGACGTTA3' (SEQ lD No 7), 5'CTGAGACTCCTTGCCATAG3' (SEQ ID No 8) for the house-kipping gene ribosome S12 used as internal control. PCR parameters were 94°C for 30 s, 52°C (for FXR and RibS12) or 60°C (for GAPDH) for 30 s, 72°C for 45 s, 25 (for GAPDH) or 30 (for FXR and RibS12) cycles after denaturing for 2 min at 94°C.

**Immunofluorescence staining** : Three days after electroporation, cells were plated in 8-well chamber slides at a density of 5x10⁴ cells per well. 24 hours after, slides were washed in PBS and fixed in 2/3 acetone-ethanol for 5 minutes at -20°C followed by 50 minutes block in PBS-glycin 0.2M. Primary antibodies were added to the cells at a 1:500 dilution in PBS-glycin 0.2M for 1 h at room temperature. After 3 washes in PBS, fluorescein conjugated secondary antibodies (Jackson ImmunoResearch) were added to the cells at a 1:50 dilution in PBS-glycin 0.2M for 40 minutes at room temperature. In the meantime, cells were counterstained with Evans Blue (Merck).

**FACS analysis :** 72h post-electroporation, single-cell suspension was prepared for FACS analysis. Briefly 0.8.10⁶ cells were fixed and permeabilised for 15 min at room temperature with Cytofix/Cytoperm (PharMingen). Cells were stained for 30 min with primary antibodies at a 1:500 dilution in Perm/Wash Buffer (PharMingen). Bound monoclonal antibody was detected by incubation for 30 min at 4°C with secondary antibody diluted 1:100 in Perm/Wash Buffer. The stained cells were resuspended in PBS prior to analysis using a FACSCalibur 3C (BD Biosciences).

### Results:

**Effects of BAs on HCV RNA replication in Huh7 and Huh7-Lunet transfected cells :** Huh7 cells were first treated with DCA, the major secondary BA found in human bile that represents around 20% of the total pool of BAs (Kullak-Ublick GA. Et al. 2004). DCA was added to the cell culture medium at a concentration of 100µM which corresponds to serum BAs concentrations usually measured during hepatitis (Fischer S. et al. 1996). The effect on replication of the genotype 1 b replicon (R1 b) was monitored every day until cells became confluent by measuring luciferase activity under the dependence of HCV RNA replicon expression. No increase in the mortality of DCA treated cells could be noted compared to the untreated ones. Four hours after electroporation, luciferase activities in replicon R1 b and replication defective control replicon (Rp-del) treated cells were very similar, reflecting the translation of the electroporated RNA and thus the transfection efficiency. Subsequently luciferase activity in Rp-del treated-cells diminished continuously and reached background levels at 72h (Figure 1) confirming that luciferase activity desappears in the absence of replication. The luciferase activity curve of DCA-treated Rp-del cells was strictly superposed to that of the untreated Rp-del cells, implying that DCA did not increase RNA stability or HCV proteins half-lifes. In R1 b-transfected cells, luciferase activity first decreased, then stabilized at 48h and remained steady at low level. By contrast luciferase activities of DCA-treated R1 b cells were significantly higher especially at 72h of DCA treatment when cells became confluent, showing a 15-fold increase. We then wanted to assess whether these high replication levels could be maintained over the time after several passages. Cells were split when they became confluent (every 3 or 4 days) and seeded back with a 1:3 dilution. BAs were added 24h after each passage to let the cells recover and to simulate natural pulses of BA secretion. High levels of luciferase activities could be maintained for more than 2 weeks (Figure 2). However, luciferase activity only slightly increased after 72h of treatment and this time point had thus been chosen in subsequent experiments.

Upon BA treatment luciferase activities varied in the same proportions in the highly permissive cell line Huh7-Lunet as in Huh7 (data not shown). We then wanted to confirm that the effect observed was a direct effect on replication. As replication rate is higher in Huh7-Lunet cells than in Huh7 cells (Fischer S. et al. 1996), those cells were used to quantify by RQ-PCR the replicative HCV RNA negative strand intermediate. As shown in Figure 3 HCV RNA levels increased in the same proportions as luciferase activities upon DCA treatment.

Altogether these data indicate that DCA-induced increase of luciferase activity results from an up-regulation of HCV RNA replication rather than from protein or RNA stabilization.

**The effect of BAs is mediated by the nuclear-receptor FXR :** Dose-response experiments were then performed with the free (hydrophobic) and conjugated (hydrosoluble) forms of the major primary and secondary BAs (CDCA and DCA respectively) (Figure 4A). BAs were added at concentrations observed in the serum of healthy individuals (around 5 to 10µM) or during chronic cholestatic hepatitis (around 100µM) (Fischer S. et al. 1996). The effect of free DCA and CDCA on luciferase activity was dose-dependent, with some specific activity already detectable at concentrations as low as 10µM for CDCA and reaching a maximum level at 100µM. Higher concentrations (above 200µM) were toxic for the replicon transfected cells (not shown). CDCA appeared to up-regulate HCV RNA replication more efficiently than DCA. Results of more than 30 similar experiments always showed a remarkably high effect of these free BAs. Luciferase activity in R1 b Huh7 or Huh7-Lunet cells treated with 100µM of CDCA were regularly between 10 to 20-fold over the basal level. On the opposite no or only a slight effect of the glyco- or tauro-conjugated derivatives could be observed. Both conjugated and free BAs bind to the plasma membrane receptor TGR5 while only free BAs can cross the plasma membrane in the absence of specific transporters which are not expressed in hepatoma cell lines (Brown MS. et al. 1997) and activate nuclear receptors. Therefore, the effect on HCV RNA replication of BAs restricted to the free BA forms suggests a mechanism mediated by nuclear receptors.

BAs, sterols and fatty acids are structurally related natural ligands of the NR1 family of nuclear receptors expressed in the liver and intestine. On the opposite of the endocrine molecules which activate the classic steroid receptors in nanomolar range, ligands of these receptors (BAs, lipids and steroids) are physiologically present at high concentration (µM range) (Francis GA. et al. 2003) and activate their receptors with EC50 of 10-15 µM (Edwards PA. et al. 2002). The concentrations of the NR1-agonists used in this study were chosen according to the study of Parks et al. (Parks DJ. et al. 1999) and were the highest concentrations that did not show cytotoxic effect on Huh7 cells after evaluation by microscopic observation and Trypan blue exclusion assay. BAs were recently described as the natural ligands for FXR (Makishima M. et al. 2002; Parks DJ. et al. 1999). To examine whether BAs were mediating their effects on HCV RNA replication through FXR we tested a panel of FXR ligands including the two formerly described FXR ligands that activate FXR at supraphysiological concentrations, farnesol and the synthetic retinoid TTNPB at a concentration of 10µM each and other BAs such as CA, LCA, and UDCA, all at a concentration of 100µM (Figure 4B). We found that CDCA was the most potent activator, followed by the secondary BAs DCA and LCA. CA and UDCA showed only weak activity. Luciferase activity was also slightly increased with farnesol and TTNPB. These results suggest that all FXR agonists are able to enhance HCV replication. The profile of activity on HCV replication is very similar to the pattern of ligands published for FXR activation (Makishima M. et al. 2002), suggesting that FXR is the receptor mediating BAs effects on HCV RNA replication in Huh7 cells.

As LCA is also a ligand for PXR we tested naturally occuring steroids including pregnenolone (10µM), progesterone (5µM), dexamethasone (a synthetic glucocorticoid) (10µM) that have been shown to activate PXR (Goodwin B. et al. 2003). None of these compounds induced any upregulation of the luciferase activity. To further exclude a possible cross-reaction with other nuclear receptors, we tested other structuraly related compounds know to bind receptors of the NR1 family like oxysterols (cholesterol (10µM), 22-hydroxycholesterol (5µM) or 25-hydroxycholesterol (1µM)), which are ligands for the liver X receptor LXR. They did not show any effect on replication either. Neither did the sole activation of the heterodimer partner of these receptors, RXR, by the 9-cis-retinoid acid.

HCV RNA replication is thus upregulated by agonists of the farnesoid nuclear receptor FXR.

**FXR inhibition decreases HCV RNA replication :** We wondered next if BAs effect on HCV RNA replication could be blocked by FXR antagonists. Guggulsterone (GGS) is a natural sterol extracted from the indian guggul tree (*Commiphora mukul*) which antagonizes FXR in the micromolar range (Urizar NL. et al. 2002). When GGS was added to the cell culture, the effect of CDCA was inhibited in a dose-dependent fashion (Figure 5A). Similar results could be obtained for cells treated with DCA (data not shown). Notably GGS could also inhibit the basal replication of HCV in the absence of BAs stimulation. This is intriguing because in a transactivation assay in HepG2 cells with a bile salt export pump promoter-driven luciferase construct, GGS alone had no effect on FXR activity (Urizar NL. et al. 2002). This supposes a basal activity of FXR in Huh7 cells that might be due to cellular BA synthesis and intracellular content of BAs. Basal level of FXR activity may be sufficient to activate other related promoters (Laffitte BA. et al. 2000) and therefore be sensitive to GGS inhibition in absence of exogenous FXR activation. The inhibitory concentration causing a decrease of half the basal activity (IC50) was around 2µM, which is within the range of a specific inhibition of FXR (Nakabayashi H. et al. 1982). 20 µM of GSS led to an almost total inhibition of the replication (up to 95%). Higher concentrations (40µM) were toxic for the replicon transfected cells.

It was recently published that GGS was also a high affinity ligand for several other steroid receptors (Burris TP. et al. 2005). We further confirmed the role of FXR in controlling HCV RNA replication by performing FXR gene-silencing experiments. 24 hours before electroporation and addition of BAs, Huh7 cells were transfected with siRNA duplexes to induce FXR or GAPDH gene silencing. As shown in Figure 5B the activity of CDCA decreased significantly (Student's t test p = 0,0001) by 77%, 3 days after the electroporation. The efficiency of siRNA was confirmed by RT-PCR analysis of FXR and GAPDH transcripts 48h after their transfection (Figure 5C). Overall, these results suggest that specific antagonism of this receptor can inhibit HCV RNA replication even without addition of exogenous BAs.

**FXR-modulators modify HCV proteins expression :** We then investigated whether the modulation of the HCV RNA replication was correlated with a modulation in protein expression. For this purpose, we looked at the effect of FXR modulators at individual cellular level by immunolabelling the nonstructural proteins NS4a and NS5a. Rp-del transfected cells were used as a negative control. In these control cells, as no replication occurs, no HCV protein could be detected after 3 days of culture. On the contrary in cells were replication occurs (ie R1b-transfected) a significant number of positive cells could be detected either with anti-NS4a or with anti-NS5a antibodies; these positive cells were quantified to 22% by FACS analysis of the NS5a-labeling. CDCA treated-cultures only showed a moderate increase in the number of positive cells (27%) but the mean fluorescence intensity in the positive cells increased substantially from 16 to 39. These results suggest mostly an increase of replication and genome expression within the infected cells rather than an increase in the propagation of replicon harbouring cells. Again GGS almost completely abolished NS4a and NS5a expression with only 4% remaining positive cells after 3 days of treatment and very weak fluorescence intensity in these cells. This suggests that FXR antagonists might help achieving clearance of HCV in infected cells.

**IFN and FXR modulators act independently on HCV RNA replication :** Since it has been suggested that BAs could modulate IFN signaling pathway by inhibiting the 2'5'oligoadenylate synthetase activity (OAS) (Podevin P. et al. 1999) and that they have been proposed to down-regulate the phosphorylation of STAT1 induced by IFN, hence allowing PEC replication (Chang KO. et al. 2004), we wondered if the up-regulation of HCV RNA replication by BAs was also caused by an interference with IFN activity in our system. To address this question we treated R1 b-transfected cells with IFN concentrations ranging from 0 to 50 U/mL in association or not with CDCA treatment.

As expected in presence of CDCA, HCV RNA replication was far much elevated than in untreated cells (Figure 6). Nevertheless, as for BA-untreated cells, IFN treatment could still inhibit gradually this replication. In both conditions IFN treatment began to be effective at 0,1 U/mL and the IC50 were very close (0,25U/mL and 0,3U/mL respectively). Therefore BAs are not affecting IFN-alpha activity. The up-regulation of HCV RNA replication by BAs is independent on the inhibition of an IFN pathway in these cells.

Moreover the association of 10µM GGS to a given concentration of IFN could further decrease luciferase activity by about 10 fold. An inhibition of 90% of the replication could be reached with 0,9U/mL of IFN in mock and CDCA treated cells. When 10µM of GGS was added to the cultures, the same level of inhibition could be reached with only 0,1 (mock) or 0,2U/mL (CDCA) of IFN. The effects of IFN and GGS appeared thus to be independent. FXR might thus be foreseen as a target for anti-HCV therapy in association to IFN either to increase the SVR rate or to reduce IFN toxic secondary effects.

**Comparative effect of FXR modulation on different HCV genotypes :** Apart from the Con1-genotype 1b replicon used so far in this study, one other replicon derived from one other genotypes has been developped. Genotype 1 a replicons from strain H77 require highly permissive cell lines such as Huh7-Lunet or Huh7.5 to replicate (Blight KJ. et al. 2002To assess whether these replicons were also susceptible to FXR-modulation, Huh7-Lunet cells were transfected in parallel with R1 b and R1 a and treated with CDCA as FXR-agonist or GGS as FXR-antagonist (Figure 7). Genotype 1 a replicon had the same behaviour as genotype 1 b in response to FXR-modulation at 72h of treatment.

### Discussion :

Treatment of cells with physiological and pathological concentrations of BAs enhanced greatly, up to more than 10 fold, the luciferase activity in cells harbouring bicistronic subgenomic HCV replicons of genotype 1 that bear the luciferase gene under the control of the 5' untranslated region of HCV. No effect of BAs was noted on the replication-defective replicon, excluding a possible stabilizing effect of the replicons by BAs such as protection against RNase. It was also observed after BA treatment a direct increase of the number of the HCV RNA negative strand, the mandatory replicative intermediate, in the same proportion as the luciferase activity variation. Replication of genotype 1 HCV subgenomic replicons appeared thus to be highly stimulated by a BA-induced pathway in Huh7 cell lines. Only the unconjugated BAs which can freely cross the plasma membrane could modulate HCV RNA replication, suggesting a mechanism dependent on a nuclear receptor. Indeed, among free BAs, only the FXR ligands were active on HCV RNA replication whereas a FXR antagonist GGS and FXR invalidation by siRNA abrogated BA-induced HCV RNA replication. Moreover even the basal level of HCV RNA replication in the absence of exogenous BA was inhibited by GGS suggesting that the basal cellular level of activated FXR is necessary for maintaining HCV RNA replication.

These results identify a BA signaling pathway dependent on FXR that is necessary for at least genotype 1 HCV RNA replication. To further characterize this pathway, we asked for a possible relationship of this pathway with the interferon type 1 antiviral activity. Indeed activation of the plasma membrane receptor TGR5 by both conjugated and unconjugated BAs activate the mitogen-activated protein kinase pathway that increases intracellular cAMP level and inhibits the phosphorylation of STAT1 by type 1 IFN (Chang KO. et al. 2004). BAs were shown to be necessary in vitro for the replication of the porcine enteric virus through the stimulation of TGR5. However, in the present study, the absence of effect of conjugated BAs rendered unlikely a role of TGR5 in controlling HCV RNA replication. It has also been proposed that BAs, and more specifically unconjugated CDCA, could modulate different steps of the IFN signaling pathway in hepatoma cell lines, inhibiting the induction of expression and the activation of OAS, MxA and the RNA-activated protein kinase PKR which are major proteins of the IFN response (Podevin P. et al. 1999). The effect of BAs on HCV RNA replication appeared to be independent on IFN modulation as CDCA did not inhibit IFN treatment efficacy to suppress HCV RNA replication, which was as active in CDCA-treated cells as in untreated ones. Therefore the FXR-dependent activation of HCV RNA replication does not rely on an inhibition of IFN anti-viral activity.

Cholesterol is catabolized into BAs by the cholesterol 7a-hydroxylase CYP7A1 which controls the first step of BA synthesis by the liver. Intracellular levels of BAs regulate cholesterol degradation by a negative feedback on this enzyme via two mechanisms. First BA-induced activation of FXR modulates the expression of the small heterodimeric partner (SHP) which then represses the 7a-hydroxylase CYP7A1 promoter (Goodwin B. et al. 2003). The second mechanism of the negative feedback implies the JNK pathway and is independent on SHP and FXR (Li T. et al. 2006). Blocking the degradation of cholesterol should likely increase cellular cholesterol load. High level of cholesterol in turn down-regulates the HMG CoA reductase, the first enzyme of the mevalonate pathway that produces cholesterol and the non-sterol isoprenoid products, by retaining its transactivator, SREBP-1, within the endoplasmic reticulum membrane (Brown MS. et al. 1997). In addition, BAs-induced activation of FXR also decreases expression of SREBP-1c (Watanabe M. et al. 2004). Treatment of cells with BAs would thus repress SREBP activity through several mechanisms and consequently inhibit the mevalonate pathway and lower the pool of isoprenoids including geranylgeraniol. HCV RNA replication is inhibited by statins which are HMG-CoA reductase inhibitors (Ikeda M. et al. 2006, Kapadia SB. et al. 2005, Ye J. et al. 2003). The inhibitory effect of statins on HCV RNA replication can be rescued by addition of mevalonate and more potently by geranylgeraniol supplementation. Indeed prenylation of FbI2, i.e. covalent binding of geranylgeraniol via formation of a cysteine thioesther, was shown to be necessary for HCV RNA replication (Wang C. et al. 2005). If BAs were acting through a modification of the protein prenylation, they would rather reduce the replication of HCV RNA than favouring its replication. In agreement with this hypothesis, BAs treated cells remained sensitive and were even more sensitive to the addition of geranylgeraniol than untreated cells for HCV RNA replication (data not shown). Interestingly, statins decrease the FXR expression at both the RNA and protein levels and down regulate its DNA-binding activity (Habeos I. et al. 2005). The inhibition of HCV RNA replication by statins may thus also be related in part to FXR down regulation.

Besides controlling the cholesterol and BAs synthesis pathways, FXR also regulates many other genes of the lipid and triglyceride metabolism (Kalaany NY. et al. 2006). Modification of these intracellular lipids may modify the cellular membrane lipid composition. Such modification may favour HCV replication as some groups have demonstrated that HCV RNA replication occurs within lipid rafts domains that are membrane micro-domains enriched in cholesterol and sphingolipids (Aizaki H. et al. 2004). Interestingly and recently, it was shown that BAs-dependent activation of FXR is required for normal liver regeneration (Huang W. et al. 2006). Elevated BA levels accelerate liver regeneration and decreased levels inhibit liver growth after partial hepatectomy. It was proposed that FXR promotes homeostasis not only by regulating expression of appropriate metabolic target genes but also by driving homeotrophic liver growth. As HCV replication is dependent on cell multiplication (Pietschmann T. et al. 2001), BAs might also stimulate replication of HCV by promoting liver growth through FXR activation.

BAs are natural ligands found at high concentrations in the liver. Among the BAs tested CDCA showed the greatest activity on HCV RNA replication. Levels of BAs, especially those of the primary BA CDCA, are enhanced in chronic hepatitis. In these livers CDCA account for 60% of the total BAs (Fischer S. et al. 1996). Patients with high levels of BAs especially those with pruritus have a poor response to antiviral therapy (Jorquera F. et al. 2005, Lebovics E. et al. 1997). It was proposed that this defect might partly be explained by an immunosuppressive effect of CDCA on natural killer cells (Hirata M. et al. 2002). The BA-induced activation of FXR dependent stimulation of HCV RNA provides a more direct explanation to the negative predictive value of high levels of BAs to SVR to treatment. The negative predictive value of BAs does not seem to be correlated to particular HCV genotypes (Jorquera F. et al. 2005).

It has been suggested that enterocytes are a reservoir and replication site for HCV (Blight KJ. et al. 2002, Deforges S. et al. 2004). The entero-hepatic circulation of BAs exposes enterocytes to high BA concentrations. As FXR is also expressed in intestine (Forman BM. et al. 1995), the FXR-dependent BA signaling pathway that stimulates HCV RNA replication further strengthens the hypothesis that intestine contributes to the HCV plasma viral load particularly for circulating virus associated with chylomicron like particles (Diaz O. et al. 2006).

This study has thus demonstrated that FXR is critical for high HCV RNA replication at least in Huh7 cells and for genotype 1 replicons. FXR may thus be foreseen as a therapeutic target for anti-HCV therapy particularly for patients infected with genotypes 1 which profit of high BA levels for their replication. In addition, as the FXR-dependent stimulation of HCV RNA replication appeared to be independent on the action of IFN, antagonists of FXR could thus be used either to lower doses of IFN and therefore to decrease deleterious secondary effects of IFN or to increase the rate of SVR after standard therapy protocols.

### References :

1999. Global surveillance and control of hepatitis C. Report of a WHO Consultation organized in collaboration with the Viral Hepatitis Prevention Board, Antwerp, Belgium. J Viral Hepat 6:35-47.
Aizaki, H., K. J. Lee, V. M. Sung, H. Ishiko, and M. M. Lai. 2004. Characterization of the hepatitis C virus RNA replication complex associated with lipid rafts. Virology 324:450-61.
Aizaki, H., K.-J. Lee, V. M.-H. Sung, H. Ishiko, and M. M. C. Lai. 2004. Characterization of the hepatitis C virus RNA replication complex associated with lipid rafts. Virology 324:450-461.
Andre, P., F. Komurian-Pradel, S. Deforges, M. Perret, J. L. Berland, M. Sodoyer, S. Pol, C. Brechot, G. Paranhos-Baccala, and V. Lotteau. 2002. Characterization of low- and very-low-density hepatitis C virus RNA-containing particles. J Virol 76:6919-28.
Andre, P., G. Perlemuter, A. Budkowska, C. Brechot, and V. Lotteau. 2005. Hepatitis C virus particles and lipoprotein metabolism. Semin Liver Dis 25:93-104.
Blight, K. J., J. A. McKeating, and C. M. Rice. 2002. Highly permissive cell lines for subgenomic and genomic hepatitis C virus RNA replication. J Virol 76:13001-14.
Brown, M. S., and J. L. Goldstein. 1997. The SREBP pathway: regulation of cholesterol metabolism by proteolysis of a membrane-bound transcription factor. Cell 89:331-40.
Burris, T. P., C. Montrose, K. A. Houck, H. E. Osborne, W. P. Bocchinfuso, B. C. Yaden, C. C. Cheng, R. W. Zink, R. J. Barr, C. D. Hepler, V. Krishnan, H. A. Bullock, L. L. Burris, R. J. Galvin, K. Bramlett, and K. R. Stayrook. 2005. The hypolipidemic natural product guggulsterone is a promiscuous steroid receptor ligand. Mol Pharmacol 67:948-54.
Chang, K. O., S. V. Sosnovtsev, G. Belliot, Y. Kim, L. J. Saif, and K. Y. Green. 2004. Bile acids are essential for porcine enteric calicivirus replication in association with down-regulation of signal transducer and activator of transcription 1. Proc Natl Acad Sci U S A 101:8733-8.
Dhanak D, Duffy KJ, Johnston VK, Lin-Goerke J, Darcy M, Shaw AN, Gu B, Silverman C, Gates AT, Nonnemacher MR, Earnshaw DL, Casper DJ, Kaura A, Baker A, Greenwood C, Gutshall LL, Maley D, DeIVecchio A, Macarron R, Hofmann GA, Alnoah Z, Cheng HY, Chan G, Khandekar S, Keenan RM, Sarisky RT. Identification and biological characterization of heterocyclic inhibitors of the hepatitis C virus RNA-dependent RNA polymerase. J Biol Chem. 2002 Oct 11;277(41):38322-7.
Deforges, S., A. Evlashev, M. Perret, M. Sodoyer, S. Pouzol, J. Y. Scoazec, B. Bonnaud, O. Diaz, G. Paranhos-Baccala, V. Lotteau, and P. Andre. 2004. Expression of hepatitis C virus proteins in epithelial intestinal cells in vivo. J Gen Virol 85:2515-23.
Diaz, O., F. Delers, M. Maynard, S. Demignot, F. Zoulim, J. Chambaz, C. Trepo, V. Lotteau, and P. Andre. 2006. Preferential association of Hepatitis C virus with apolipoprotein B48-containing lipoproteins. J.Gen.Virol. in press.
Duran-Sandoval, D., B. Cariou, J. C. Fruchart, and B. Staels. 2005. Potential regulatory role of the farnesoid X receptor in the metabolic syndrome. Biochimie 87:93-8.
Edwards, P. A., H. R. Kast, and A. M. Anisfeld. 2002. BAREing it all: the adoption of LXR and FXR and their roles in lipid homeostasis. J Lipid Res 43:2-12.
Feld, J. J., and J. H. Hoofnagle. 2005. Mechanism of action of interferon and ribavirin in treatment of hepatitis C. Nature 436:967-72.
Ferrieu-Weisbuch C., Bettsworth F., Becquart L., Paranhos-Baccala G., Michel S., Arnaud M., Jolivet-Reynaud C. Usefulness of the phage display technology for the identification of a hepatitis C virus NS4A epitope recognized early in the course of the disease. J Virol Methods. 2006 Feb ;131:175-83)
Fischer, S., U. Beuers, U. Spengler, F. M. Zwiebel, and H. G. Koebe. 1996. Hepatic levels of bile acids in end-stage chronic cholestatic liver disease. Clin Chim Acta 251:173-86.
Forman, B. M., E. Goode, J. Chen, A. E. Oro, D. J. Bradley, T. Perlmann, D. J. Noonan, L. T. Burka, T. McMorris, W. W. Lamph, R. M. Evans, and C. Weinberger. 1995. Identification of a nuclear receptor that is activated by farnesol metabolites. Cell 81:687-93.
Francis, G. A., E. Fayard, F. Picard, and J. Auwerx. 2003. Nuclear receptors and the control of metabolism. Annu Rev Physiol 65:261-311.
Gale MJ, Korth MJ, Tang NM et al. (1997) Evidence that hepatitis C virus resistance to interferon is mediated through repression of the PKR protein kinase by the nonstructural 5A protein. Virology 230: 217-227
Goodwin, B., K. C. Gauthier, M. Umetani, M. A. Watson, M. I. Lochansky, J. L. Collins, E. Leitersdorf, D. J. Mangelsdorf, S. A. Kliewer, and J. J. Repa. 2003. Identification of bile acid precursors as endogenous ligands for the nuclear xenobiotic pregnane X receptor. Proc Natl Acad Sci U S A 100:223-8.
Habeos, I., P. G. Ziros, A. Psyrogiannis, A. G. Vagenakis, and A. G. Papavassiliou. 2005. Statins and transcriptional regulation: the FXR connection. Biochem Biophys Res Commun 334:601-5.
Hirata, M., Y. Harihara, Y. Kita, S. Saito, M. Nishimuraj, H. Yoshino, K. Sano, M. Ito, K. Kusaka, H. Kawarasaki, K. Hashizume, and M. Makuuchi. 2002. Immunosuppressive effect of chenodeoxycholic acid on natural killer cell activity in patients with biliary atresia and hepatitis C virus-related liver cirrhosis. Dig Dis Sci 47:1100-6.
Huang, W., K. Ma, J. Zhang, M. Qatanani, J. Cuvillier, J. Liu, B. Dong, X. Huang, and D. D. Moore. 2006. Nuclear receptor-dependent bile acid signaling is required for normal liver regeneration. Science 312:233-6.
Ikeda, M., K. Abe, M. Yamada, H. Dansako, K. Naka, and N. Kato. 2006. Different anti-HCV profiles of statins and their potential for combination therapy with interferon. Hepatology 44:117-25.
Jorquera, F., M. J. Monte, J. Guerra, S. Sanchez-Campos, J. A. Merayo, J. L. Olcoz, J. Gonzalez-Gallego, and J. J. Marin. 2005. Usefulness of combined measurement of serum bile acids and ferritin as additional prognostic markers to predict failure to reach sustained response to antiviral treatment in chronic hepatitis C. J Gastroenterol Hepatol 20:547-54.
Kalaany, N. Y., and D. J. Mangelsdorf. 2006. LXRS and FXR: the yin and yang of cholesterol and fat metabolism. Annu Rev Physiol 68:159-91.
Kapadia, S. B., and F. V. Chisari. 2005. Hepatitis C virus RNA replication is regulated by host geranylgeranylation and fatty acids. Proc Natl Acad Sci U S A 102:2561-6.
Kato, N., K. Sugiyama, K. Namba, H. Dansako, T. Nakamura, M. Takami, K. Naka, A. Nozaki, and K. Shimotohno. 2003. Establishment of a hepatitis C virus subgenomic replicon derived from human hepatocytes infected in vitro. Biochemical and Biophysical Research Communications 306:756-766.
Kawamata, Y., R. Fujii, M. Hosoya, M. Harada, H. Yoshida, M. Miwa, S. Fukusumi, Y. Habata, T. Itoh, Y. Shintani, S. Hinuma, Y. Fujisawa, and M. Fujino. 2003. A G protein-coupled receptor responsive to bile acids. J Biol Chem 278:9435-40.
Komurian-Pradel, F., M. Perret, B. Deiman, M. Sodoyer, V. Lotteau, G. Paranhos-Baccala, and P. Andre. 2004. Strand specific quantitative real-time PCR to study replication of hepatitis C virus genome. J Virol Methods 116:103-6.
Kronke J, Kittler R, Buchholz F, Windisch MP, Pietschmann T, Bartenschlager R, Frese M. Alternative approaches for efficient inhibition of hepatitis C virus RNA replication by small interfering RNAs. J Virol. 2004 Apr;78(7):3436-46.
Krieger, N., V. Lohmann, and R. Bartenschlager. 2001. Enhancement of hepatitis C virus RNA replication by cell culture-adaptive mutations. J Virol 75:4614-24.
Kullak-Ublick, G. A., B. Stieger, and P. J. Meier. 2004. Enterohepatic bile salt transporters in normal physiology and liver disease. Gastroenterology 126:322-42.
Laffitte, B. A., H. R. Kast, C. M. Nguyen, A. M. Zavacki, D. D. Moore, and P. A. Edwards. 2000. Identification of the DNA binding specificity and potential target genes for the farnesoid X-activated receptor. J Biol Chem 275:10638-47.
Lebovics, E., F. Seif, D. Kim, A. Elhosseiny, B. M. Dworkin, A. Casellas, S. Clark, and W. S. Rosenthal. 1997. Pruritis in chronic hepatitis C: association with high serum bile acids, advanced pathology, and bile duct abnormalities. Dig Dis Sci 42:1094-9.
Li, T., A. Jahan, and J. Y. Chiang. 2006. Bile acids and cytokines inhibit the human cholesterol 7 alpha-hydroxylase gene via the JNK/c-jun pathway in human liver cells. Hepatology 43:1202-10.
Lohmann V, Hoffmann S, Herian U, Penin F, Bartenschlager R. Viral and cellular determinants of hepatitis C virus RNA replication in cell culture. J Virol. 2003 Mar;77(5):3007-19.
Makishima, M., A. Y. Okamoto, J. J. Repa, H. Tu, R. M. Learned, A. Luk, M. V. Hull, K. D. Lustig, D. J. Mangelsdorf, and B. Shan. 1999. Identification of a nuclear receptor for bile acids. Science 284:1362-5.
Makishima, M., T. T. Lu, W. Xie, G. K. Whitfield, H. Domoto, R. M. Evans, M. R. Haussler, and D. J. Mangelsdorf. 2002. Vitamin D receptor as an intestinal bile acid sensor. Science 296:1313-6.
Maruyama, T., Y. Miyamoto, T. Nakamura, Y. Tamai, H. Okada, E. Sugiyama, T. Nakamura, H. Itadani, and K. Tanaka. 2002. Identification of membrane-type receptor for bile acids (M-BAR). Biochem Biophys Res Commun 298:714-9.
Nakabayashi, H., K. Taketa, K. Miyano, T. Yamane, and J. Sato. 1982. Growth of human hepatoma cells lines with differentiated functions in chemically defined medium. Cancer Res 42:3858-63.
Parks, D. J., S. G. Blanchard, R. K. Bledsoe, G. Chandra, T. G. Consler, S. A. Kliewer, J. B. Stimmel, T. M. Willson, A. M. Zavacki, D. D. Moore, and J. M. Lehmann. 1999. Bile acids: natural ligands for an orphan nuclear receptor. Science 284:1365-8.
Pietschmann, T., V. Lohmann, G. Rutter, K. Kurpanek, and R. Bartenschlager. 2001. Characterization of cell lines carrying self-replicating hepatitis C virus RNAs. J Virol 75:1252-64.
Podevin, P., O. Rosmorduc, F. Conti, Y. Calmus, P. J. Meier, and R. Poupon. 1999. Bile acids modulate the interferon signalling pathway. Hepatology 29:1840-7.
Quinkert, D., R. Bartenschlager, and V. Lohmann. 2005. Quantitative analysis of the hepatitis C virus replication complex. J Virol 79:13594-605.
Ramalho, F. 2003. Hepatitis C virus infection and liver steatosis. Antiviral Res 60:125-7
Shi, S. T., S. J. Polyak, H. Tu, D. R. Taylor, D. R. Gretch, and M. M. Lai. 2002. Hepatitis C virus NS5A colocalizes with the core protein on lipid droplets and interacts with apolipoproteins. Virology 292:198-210.
Su, A. I., J. P. Pezacki, L. Wodicka, A. D. Brideau, L. Supekova, R. Thimme, S. Wieland, J. Bukh, R. H. Purcell, P. G. Schultz, and F. V. Chisari. 2002. Genomic analysis of the host response to hepatitis C virus infection. Proc Natl Acad Sci U S A 99:15669-74.
Urizar NL, Moore DD. GUGULIPID: a natural cholesterol-lowering agent. Annu Rev Nutr. 2003;23:303-13. Epub 2003 Feb 26.
Urizar, N. L., A. B. Liverman, D. T. Dodds, F. V. Silva, P. Ordentlich, Y. Yan, F. J. Gonzalez, R. A. Heyman, D. J. Mangelsdorf, and D. D. Moore. 2002. A natural product that lowers cholesterol as an antagonist ligand for FXR. Science 296:1703-6.
van den Hoff, M. J., A. F. Moorman, and W. H. Lamers. 1992. Electroporation in 'intracellular' buffer increases cell survival. Nucleic Acids Res 20:2902.
Wang, C., M. Gale, Jr., B. C. Keller, H. Huang, M. S. Brown, J. L. Goldstein, and J. Ye. 2005. Identification of FBL2 as a geranylgeranylated cellular protein required for hepatitis C virus RNA replication. Mol Cell 18:425-34.
Watanabe, M., S. M. Houten, L. Wang, A. Moschetta, D. J. Mangelsdorf, R. A. Heyman, D. D. Moore, and J. Auwerx. 2004. Bile acids lower triglyceride levels via a pathway involving FXR, SHP, and SREBP-1c. J Clin Invest 113:1408-18.
Windisch, M. P., M. Frese, A. Kaul, M. Trippler, V. Lohmann, and R. Bartenschlager. 2005. Dissecting the interferon-induced inhibition of hepatitis C virus replication by using a novel host cell line. J Virol 79:13778-93.
Wu J, Xia C, Meier J, Li S, Hu X, Lala DS. 2002. The hypolipidemic natural product guggulsterone acts as an antagonist of the bile acid receptor. Mol. Endocrinol. 16:1590-97
Ye, J., C. Wang, R. Sumpter, Jr., M. S. Brown, J. L. Goldstein, and M. Gale, Jr. 2003. Disruption of hepatitis C virus RNA replication through inhibition of host protein geranylgeranylation. Proc Natl Acad Sci U S A 100:15865-70.
Zhong W, An H, Barawkar D, Hong Z. Dinucleotide analogues as novel inhibitors of RNA-dependent RNA polymerase of hepatitis C Virus. Antimicrob Agents Chemother. 2003 Aug;47(8):2674-81.

## Claims

1. Use of an antagonist of farnesoid X receptor (FXR) for the manufacture of a medicament intended for the treatment of an infection by members of the *Flaviviridae* family in a subject in need thereof.

2. Use of an antagonist of FXR for the manufacture of a medicament intended for the treatment of an HCV infection or for the treatment of a disease associated with an HCV infection in a subject in need thereof, such as acute or chronic hepatitis C or for the prevention of liver diseases, such as liver fibrosis, liver cirrhosis or hepatocellular carcinoma.

3. Use according to claim **1** or **2** wherein said antagonist is 4,17(20)-trans-pregnadiene-3,16-dione or 4,17(20)-cis-pregnadiene-3,16-dione.

4. Use of an inhibitor of FXR expression for the manufacture of a medicament intended for the treatment of an infection by members of the *Flaviviridae* family in a subject in need thereof.

5. Use of an inhibitor of FXR expression for the manufacture of a medicament intended for the treatment of an HCV infection or for the treatment of a disease associated with an HCV infection in a subject in need thereof, such as acute or chronic hepatitis C or for the prevention of liver diseases, such as liver fibrosis, liver cirrhosis or hepatocellular carcinoma.

6. Use according to claim **4** or **5** wherein said inhibitor of FXR expression is a siRNA, an antisense oligonucleotide or a ribozyme.

7. Use according to any one of claims **1** to **6** for the treatment of a subject undergoing a treatment with interferon-alpha.

8. Use according to any one of claims **1** to **7** for the treatment of a subject undergoing a treatment with a nucleoside analog.

9. Use according to any one of claims **1** to **8** for the treatment of a subject undergoing a treatment with an inhibitor of HCV proteases and/or polymerases.

10. Kit for the treatment of an infection by members of the *Flaviviridae* family or for the treatment of an HCV infection or for the treatment of a disease associated with an HCV infection, comprising a medicament comprising an antagonist of FXR or an inhibitor of FXR expression and at least a medicament selected from the group consisting of a medicament comprising interferon-alpha, a medicament comprising a nucleoside analog and a medicament comprising an inhibitor of HCV proteases and/or polymerases.

11. A cell culture system allowing the replication of HCV, comprising a culture medium for FXR expressing cells, FXR expressing cells and at least one agonist of FXR.

12. The cell culture system according to claim **11**, wherein the at least one agonist of FXR is chenodeoxycholic acid, deoxycholic acid, lithocholic acid, dehydrocholic acid, ursodeoxycholic acid, cholic acid, farnesol or (E)-[(tetrahydrotetramethylnaphthalenyl)propenyl]benzoic acid.

13. The cell culture system according to any one of claims **11** to **12** wherein said FXR expressing cells are chosen among cell monolayers of the human hepatoma cell line Huh7 or Huh7-Lunet or HepG2.

14. The cell culture system according to any one of claims **11** to **13** wherein said FRX expressing cell is a cell that has been transfected with the gene encoding FXR.

15. The cell culture system according to any one of claims **11** to **14** further comprising HCV viruses.

16. The cell culture system according to any one of claims **11** to **15**, wherein the FXR expressing cells are cells transfected with replicative HCV viral materials.

17. Use of the cell culture system according to any one of claim **11** to **16**, for diagnosing HCV infections, or screening of anti-viral compounds, or producing HCV viral particles or HCV viral proteins or producing anti-HCV vaccines.

18. An in vitro method for diagnosing an HCV infection in a subject wherein said method comprises the steps consisting of :
a) providing a culture of FXR expressing cells
b) incubating said culture of FXR expressing cells with a biological sample obtained from the subject,
c) incubating said culture of FXR expressing cells with at least one agonist of FXR prior to, after or simultaneously with step (b).
d) culturing said cells for a time sufficient for permitting HCV replication
e) detecting the level of HCV replication
wherein the detection of an HCV replication is indicative that said subject is infected with HCV.

19. A method according to claim **18** wherein said biological sample is derived from blood, serum, plasma or from a sample isolated during a biopsy.


SEQUENCE LISTING

<110>  INSERM

<120>  Methods, uses and compositions for modulating replication of HCV
       through the farnesoid X receptor (FXR) activation or inhibition

<130>  bep060327

<160>  8

<170>  PatentIn version 3.3

<210>  1
<211>  41
<212>  DNA
<213>  artificial

<220>
<223>  primer tag-RC1

<400>  1
ggccgtcatg gtggcgaata agtctagcca tggcgttagt a                          41


<210>  2
<211>  20
<212>  DNA
<213>  artificial

<220>
<223>  primer RC21

<400>  2
ctcccggggc actcgcaagc                                                  20


<210>  3
<211>  23
<212>  DNA
<213>  artificial

<220>
<223>  primer FXR

<400>  3
gcagcctgaa gagtggtact ctc                                              23


<210>  4
<211>  23
<212>  DNA
<213>  artificial

<220>
<223>  primer FXR

<400>  4
cattcagcca acattcccat ctc                                              23


<210>  5
<211>  20
<212>  DNA
<213>  artificial

<220>
<223>  primer GAPDH

<400>  5
ggaaggtgaa ggtcggagtc                                                    20


<210>  6
<211>  20
<212>  DNA
<213>  artificial

<220>
<223>  primer GAPDH

<400>  6
cacaagcttc ccgttctcag                                                    20


<210>  7
<211>  19
<212>  DNA
<213>  artificial

<220>
<223>  primer house-kipping gene ribosome S12

<400>  7
ggaggtgtaa tggacgtta                                                     19


<210>  8
<211>  19
<212>  DNA
<213>  artificial

<220>
<223>  primer house-kipping gene ribosome S12

<400>  8
ctgagactcc ttgccatag                                                     19